# EUROPEAN PATENT APPLICATION

(11) **EP 4 714 453 A1**
(43) Date of publication of application: **25.03.2026**
(21) Application number: 24811072.8
(22) Date of filing: 17.05.2024
(51) Int. Cl.: A61K 31/7105, A61K 31/7125, A61K 48/00, A61P 35/00, A61P 35/02, C12N 15/10, C12N 15/11, C12N 15/113, C12N 15/87

(54) **USE OF STRUCTURE FOR NUCLEIC ACID DELIVERY IN TREATMENT OF HEMATOPOIETIC TUMOR**

(30) Priority: 19.05.2023 JP 2023082728
(71) Applicant: Japan Science and Technology Agency, Kawaguchi-shi, Saitama 332-0012 (JP)
(72) Inventor: MIYAMOTO Noriko, Toyota-shi, Aichi 470-0392 (JP); KITADE Yukio, Toyota-shi, Aichi 470-0392 (JP); AKAO Yukihiro, Higher Education and Research System 1-1, Yanagido Gifu-shi, Gifu 501-1194 (JP)
(74) Representative: Grünecker Patent- und Rechtsanwälte PartG mbB
(86) International application number: PCT/JP2024/018410
(87) International publication number: WO 2024/242065

(57) **Abstract**

The present disclosure is a nucleic acid delivery construct used for treatment of a hematologic malignancy, the nucleic acid delivery construct having an assembly structure in which a nucleic acid analog represented by the following formula (1) and a nucleic acid to be delivered containing a microRNA that regulates a network of the oncogene KRAS are assembled with each other through electrostatic interaction. Wherein N represents a cationic artificial nucleic acid, H represents a hydrophilic polymer, S1 represents a spacer 1, S2 represents a spacer 2, and L represents a ligand; N has structural units in which a base is bonded to a hexose selected from ribose and deoxyribose, linking structures linking two of the structural units, and a backbone structure having cationic groups; and the cationic artificial nucleic acid is capable of assembling through electrostatic interaction between a phosphate group of the nucleic acid to be delivered and the cationic group.

## Description

### Technical Field

The present disclosure relates to use of a nucleic acid delivery construct for treatment of a hematologic malignancy.

### Background Art

In the field of gene therapy, an oligonucleotide such as DNA or RNA is delivered to a target site such as a cell to suppress expression of a gene or recombine a gene, thereby treating a disease. In such treatment, a technology of a drug delivery system (DDS) that efficiently delivers a nucleic acid to a target site is important. Various DDS methods have been developed so far. In addition, nucleic acid analysis, diagnostic tracers, molecular machines, and molecular computers have been actively applied to the fields of nanomaterials and artificial cells.

On the other hand, for example, drug discovery targeting RAS of a cancer promoting gene assembled with 30% of all cancers has been widely advanced over the past 40 years. However, since RAS protein has a narrow small molecular pocket and it is difficult to design an inhibitor, and cancer cells acquire resistance by one genetic control of RAS by antisense-oligonucleotide (AS-ODN) or siRNA, an effective therapeutic agent has not yet been developed.

The inventors have found that a nucleic acid characterized by a structure in which a hydrophilic group is linked to the 3' end of an artificial nucleic acid composed of structural units each having a base bound to a cyclic structure selected from ribose and deoxyribose and a linking structure connecting two of the structural units forms a micelle or a nanoparticle by annealing with RNA; that the micelle or the nanoparticle functions as a vesicle for delivering RNA; and that a composition in which miR-143 is formed into nanoparticles using a cationic artificial nucleic acid is effective in suppressing the growth of colorectal cancer, which is a solid tumor (Patent Literature 1).

Hematologic malignancies, represented by leukemia, are diseases in which leukocytes or immature cells at their developmental stages proliferate uncontrollably. Hematologic malignancies are difficult to treat by surgical methods alone, unlike solid tumors. Some hematologic malignancies, particularly RAS-mutated multiple myeloma, are known as cancers that repeatedly recurrence and have a poor prognosis. The inventors have found that miR-143 and derivatives thereof are effective against RAS-mutated cancers (Patent Literature 2).

### Citation List

### Patent Literature

Patent Literature 1: WO 2022/230990 A
Patent Literature 2: WO 2017/179660 A

### Summary of Invention

### Technical Problem

Among hematologic malignancies represented by leukemia, RAS-mutated multiple myeloma is known; however, it is considered to be prone to recurrence and to have a poor prognosis. Although miR-143, which is effective against solid tumors, is also expected to be effective against such hematopoietic cancers, the introduction of nucleic acids into hematopoietic cancers composed of suspended cells has been difficult, and a novel nucleic acid drug delivery system has been required.

An object of the present disclosure is to provide a nucleic acid delivery construct and a method for producing the same, effective in the treatment of a hematologic malignancy, a pharmaceutical for a hematologic malignancy, and a method for treating a hematologic malignancy.

### Solution to Problem

The inventors have found that micelles or nanoparticles composed of a cationic artificial nucleic acid and a delivery nucleotide, whose surfaces are modified with a ligand, inhibit proliferation of cells derived from hematologic malignancies, thereby completing the present disclosure.
[1] Use of a nucleic acid delivery construct for treatment of a hematologic malignancy, the nucleic acid delivery construct having an assembly structure in which a nucleic acid analog represented by the following formula (1) and a nucleic acid to be delivered containing a microRNA that regulates a network of the oncogene KRAS are assembled with each other through electrostatic interaction:

   [Chem. 1] **L-(S2)s-H-(S1)t-N (1)**

   wherein N represents a cationic artificial nucleic acid, H represents a hydrophilic polymer, S1 represents a spacer 1, S2 represents a spacer 2, L represents a ligand, s represents 0 or 1, and t represents 0 or 1;
   N has structural units in which a base is bonded to a hexose selected from ribose and deoxyribose, linking structures linking two of the structural units, and a backbone structure having cationic groups; and
   the cationic artificial nucleic acid is capable of assembling through electrostatic interaction between a phosphate group of the nucleic acid to be delivered and the cationic group.
[2] The use of a nucleic acid delivery construct for treatment of a hematologic malignancy according to [1], in which a pKa of the cationic group is within a range of 6 to 9.
[3] The use of a nucleic acid delivery construct for treatment of a hematologic malignancy according to any one of [1] and [2], in which the cationic group, in a cationic state, has a partial structure selected from the group consisting of the following formulas (C1) to (C7): 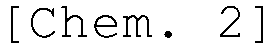 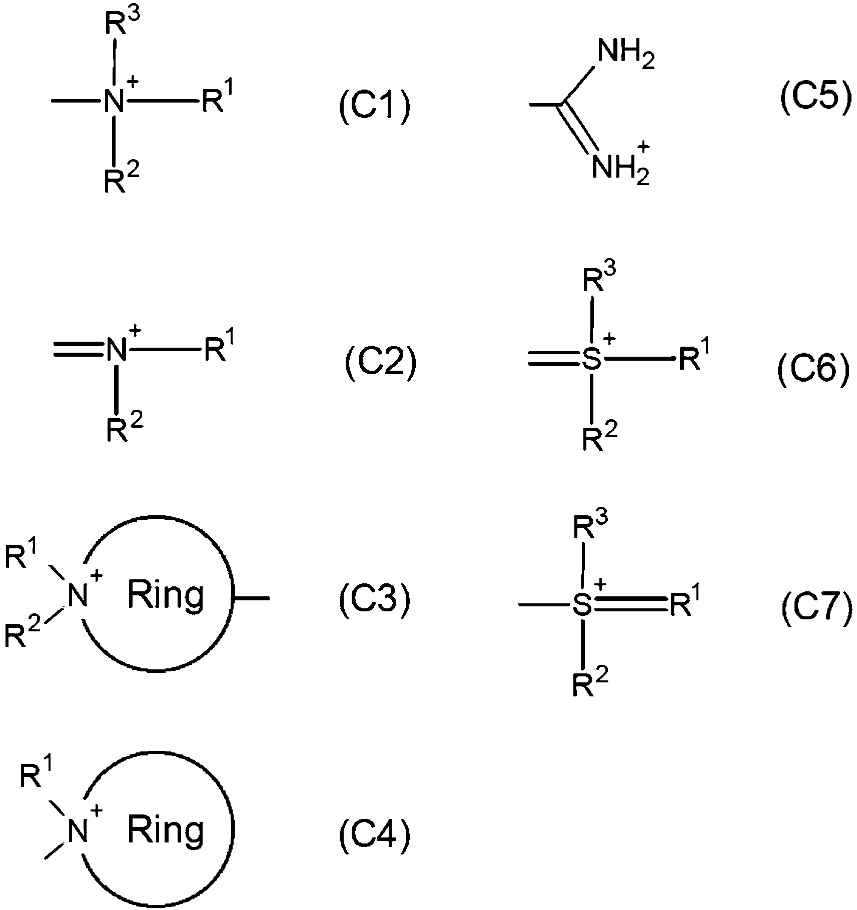 wherein R¹ to R³ represent hydrogen or an alkyl group having 1 to 10 carbon atoms, R¹ to R³ may be the same as or different from each other, and Ring is a cyclic compound including 4 to 8 carbon atoms, and may be a heterocyclic ring in which one or more of the carbon atoms are substituted with a heteroatom selected from nitrogen, oxygen, and sulfur.
[4] The use of a nucleic acid delivery construct for treatment of a hematologic malignancy according to any one of [1] to [3], in which the linking structure of the cationic artificial nucleic acid (N), in a cationic state, has at least a structure selected from the following formulas (L1) to (L4): 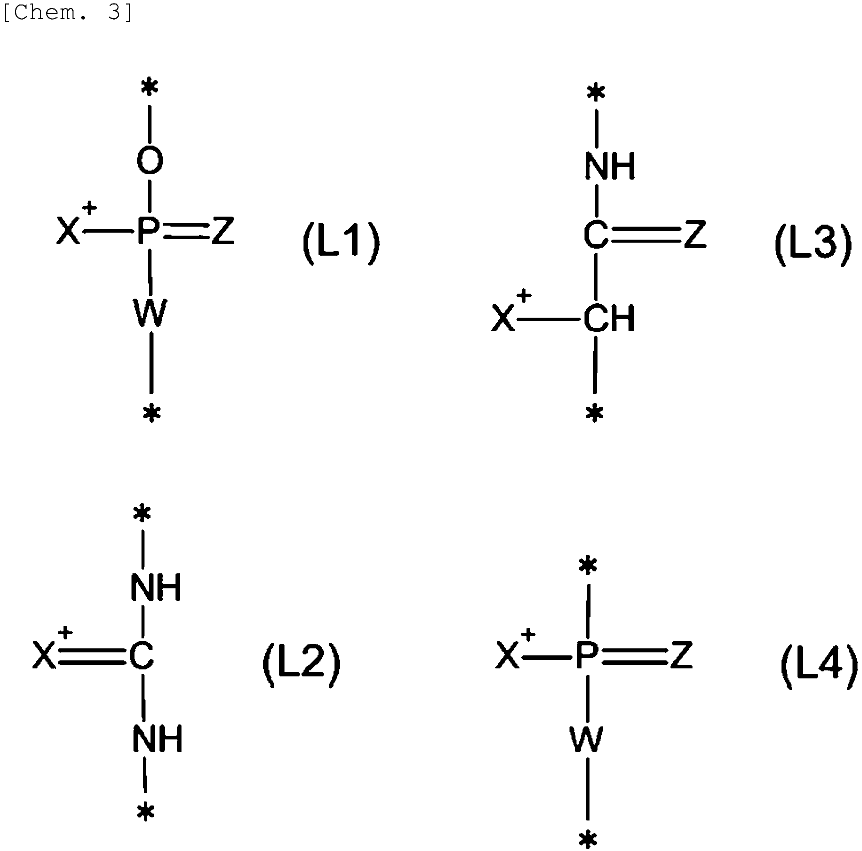 wherein X⁺ is a functional group containing the cationic group, Z represents O or S, W represents -O- or -NR⁴-, wherein R⁴ represents hydrogen or an alkyl group having 1 to 10 carbon atoms, and * denotes a bond with the structural unit adjacent thereto.
[5] The use of a nucleic acid delivery construct for treatment of a hematologic malignancy according to any one of [1] to [4], in which the cationic artificial nucleic acid has a nucleotide backbone represented by the following formula (N1): 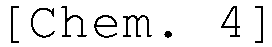 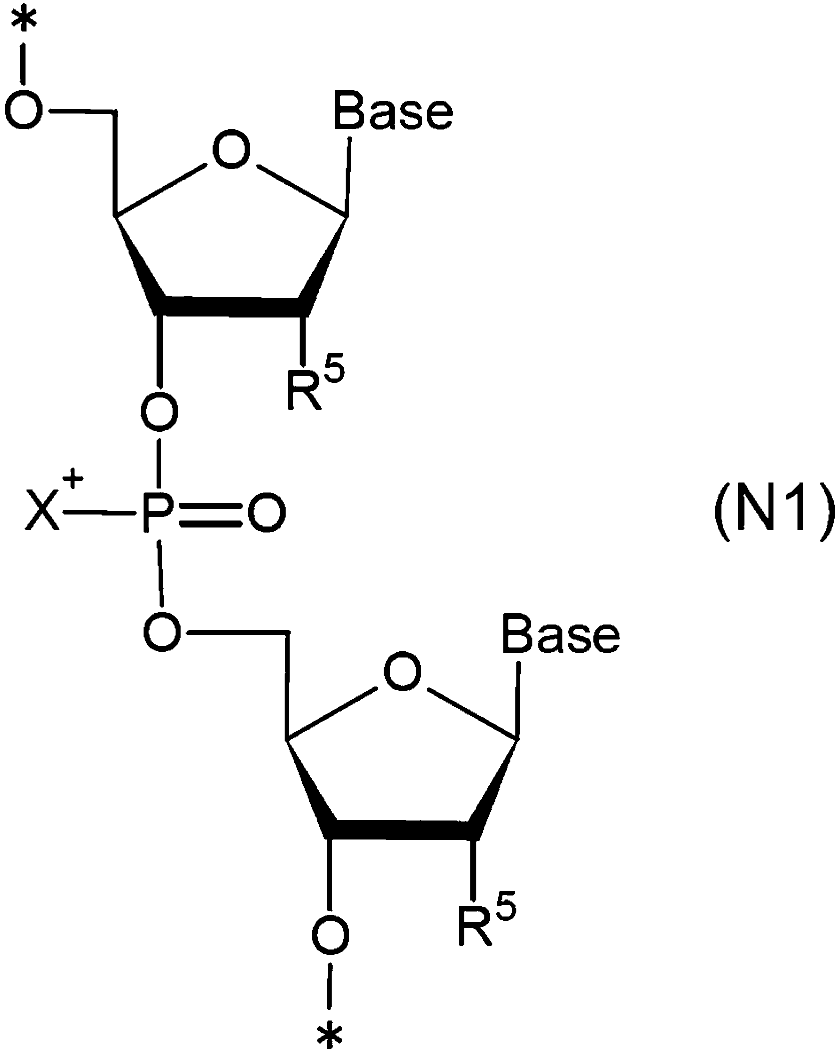 wherein X⁺ is a functional group containing the cationic group, Base represents a base, R⁵ represents H or OH, * denotes a bond with a phosphate of an adjacent nucleotide backbone, at least one of * is bonded to the hydrophilic polymer at the 5' end or 3' end, and when not bonded to the hydrophilic polymer, * is hydrogen.
[6] The use of a nucleic acid delivery construct for treatment of a hematologic malignancy according to [5], in which X⁺, in a cationic state, is capable of being an ammonium cation represented by the following formula (F1): 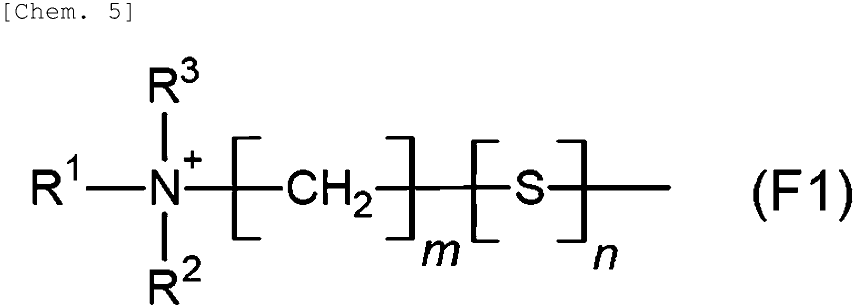 wherein R¹ to R³ represent hydrogen or an alkyl group having 1 to 10 carbon atoms, and may be the same as or different from each other, m represents an integer between 0 and 10, and n represents an integer of 0 or 1.
[7] The use of a nucleic acid delivery construct for treatment of a hematologic malignancy according to [1], in which the hydrophilic polymer is selected from polyethylene glycol, polyvinyl alcohol, polyglutamic acid, polyvinylpyrrolidone, polyacrylamide, polyethyleneimine, polyalkyl acrylate, polyoxazoline, polyacrylamide, poly(carboxybetaine methacrylate), poly(sulfobetaine methacrylate), poly(2-methacryloyloxyethyl phosphocholine), hyaluronic acid, chitosan, dextran, and derivatives thereof.
[8] The use of a nucleic acid delivery construct for treatment of a hematologic malignancy according to [7], in which the hydrophilic polymer has a polyethylene glycol backbone represented by the following formula (A1): 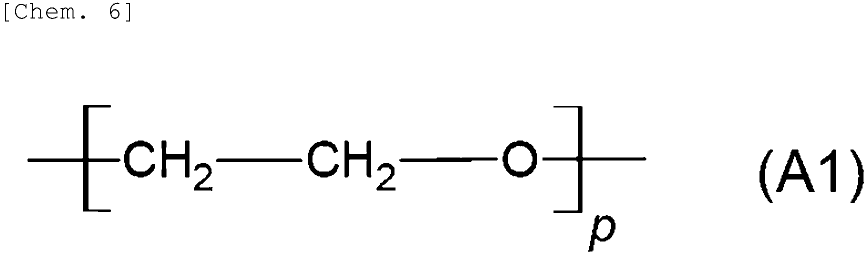 wherein p represents an integer between 1 and 20.
[9] The use of a nucleic acid delivery construct for treatment of a hematologic malignancy according to [8], in which the formula (A1) is linked through a phosphodiester group.
[10] The use of a nucleic acid delivery construct for treatment of a hematologic malignancy according to any one of [1] to [9], in which the spacer 1 (S1) has a bond represented by the following formula (S11):

   [Chem. 7] **-O-R⁶-S-S-R⁷-O- (S11)**

   wherein R⁶ and R⁷ in the formula represent a methylene group having 1 to 12 carbon atoms, and R⁶ and R⁷ may be the same as or different from each other.
[11] The use of a nucleic acid delivery construct for treatment of a hematologic malignancy according to any one of [1] to [10], in which the spacer 2 (S2) is a phosphodiester bond or a phosphodiester bond including a triazole represented by the following formula (S21): 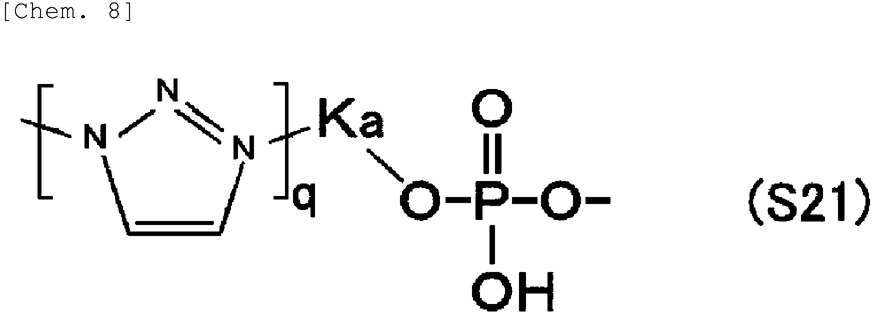 in the formula, Ka represents an amide bond containing a methylene group having 1 to 20 carbon atoms, a compound containing an aromatic ring having 6 to 12 carbon atoms, or a direct bond, q is 0 or 1, and when q is 0, the 1,2,3-triazole ring is bound to the ligand.
[12] The use of a nucleic acid delivery construct for treatment of a hematologic malignancy according to any one of [1] to [11], in which the ligand (L) is selected from glucose, mannose, galactose, sucrose, maltose, and lactose.
[13] The use of a nucleic acid delivery construct for treatment of a hematologic malignancy according to any one of [1] to [12], in which the nucleic acid delivery construct is a nanoscale construct in which a plurality of nucleic acid delivery constructs are assembled with each other.
[14] The use of a nucleic acid delivery construct for treatment of a hematologic malignancy according to [13], in which the nucleic acid delivery construct is a vesicle or a micelle in which the assembly structure is located on an inner side and the hydrophilic polymer and the ligand are located on an outer side.
[15] The use of a nucleic acid delivery construct for treatment of a hematologic malignancy according to any one of [1] to [14], in which the nucleic acid to be delivered is selected from miR-143 or an analog thereof.
[16] The use of a nucleic acid delivery construct for treatment of a hematologic malignancy according to [15], in which the miR-143 is selected from the group consisting of SEQ-1 to SEQ-23.
[17] Use of a pharmaceutical for treatment of a hematologic malignancy, the pharmaceutical including the nucleic acid delivery construct according to any one of [1] to [16].
[18] A method for treating a hematologic malignancy, the method including administering the nucleic acid delivery construct according to any one of [1] to [16] to a patient.
[19] A method for inhibiting proliferation of cancerous cells in a hematologic malignancy, the method including administering, to the cancerous cells, a nucleic acid delivery construct having an assembly structure in which a nucleic acid analog represented by the following formula (1) and a nucleic acid to be delivered containing a microRNA that regulates a network of the oncogene KRAS are assembled with each other through electrostatic interaction:

   [Chem. 9] **L-(S2)s-H-(S1)t-N (1)**

   wherein N represents a cationic artificial nucleic acid, H represents a hydrophilic polymer, S1 represents a spacer 1, S2 represents a spacer 2, L represents a ligand, s represents 0 or 1, and t represents 0 or 1;
   N has structural units in which a base is bonded to a hexose selected from ribose and deoxyribose, linking structures linking two of the structural units, and a backbone structure having cationic groups; and
   the cationic artificial nucleic acid is capable of assembling through electrostatic interaction between a phosphate group of the nucleic acid to be delivered and the cationic group.
[20] A nucleic acid delivery construct used for treatment of a hematologic malignancy, the nucleic acid delivery construct having an assembly structure in which a nucleic acid analog represented by the following formula (1) and a nucleic acid to be delivered containing a microRNA that regulates a network of the oncogene KRAS are assembled with each other through electrostatic interaction:

   [Chem. 10] **L-(S2)s-H-(S1)t-N (1)**

   wherein N represents a cationic artificial nucleic acid, H represents a hydrophilic polymer, S1 represents a spacer 1, S2 represents a spacer 2, L represents a ligand, s represents 0 or 1, and t represents 0 or 1;
   N has structural units in which a base is bonded to a hexose selected from ribose and deoxyribose, linking structures linking two of the structural units, and a backbone structure having cationic groups; and
   the cationic artificial nucleic acid is capable of assembling through electrostatic interaction between a phosphate group of the nucleic acid to be delivered and the cationic group.

### Advantageous Effects of Invention

According to the present disclosure, it is possible to provide a nucleic acid delivery construct effective in the treatment of a hematologic malignancy, a method for producing the same, a pharmaceutical for a hematologic malignancy, and a method for treating a hematologic malignancy.

### Brief Description of Drawings

Fig. 1 is a diagram illustrating an outline of a nucleic acid analog and a nucleic acid delivery construct of the present disclosure. (a) is a diagram illustrating a sequence design for Glu-RION-miR143#12. (b) is a diagram illustrating generation of a construct for delivery of Glu-RION-miR143#12. (c) is a diagram illustrating a particle size distribution of Glu-RION-miR143#12. (d) is a diagram illustrating the stability in blood after administration of Glu-RION-miR143#12 to mice.
Fig. 2 is a diagram illustrating an example of a synthesis scheme of a cationic artificial nucleic acid.
Fig. 3 is a diagram illustrating an example of a synthetic scheme.
Fig. 4 is a diagram illustrating cell viability in DLD-1 cells after 48-hour treatment with miR-143#12 using RION (control) and Glu-RION. No inhibitory effect was observed in the control.

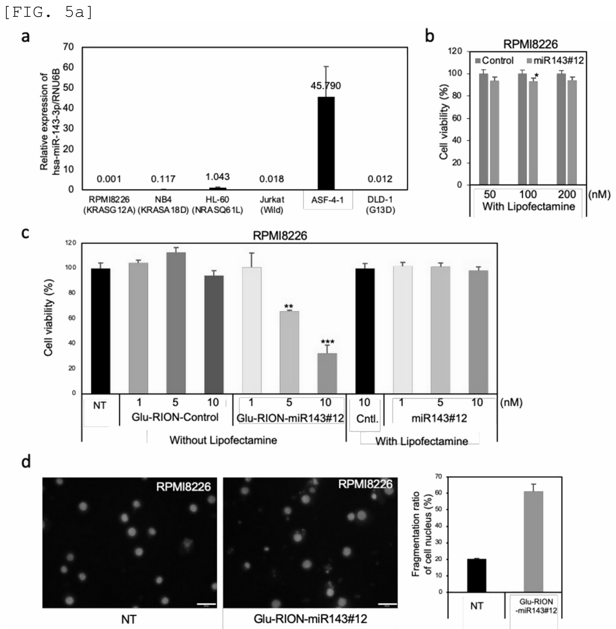

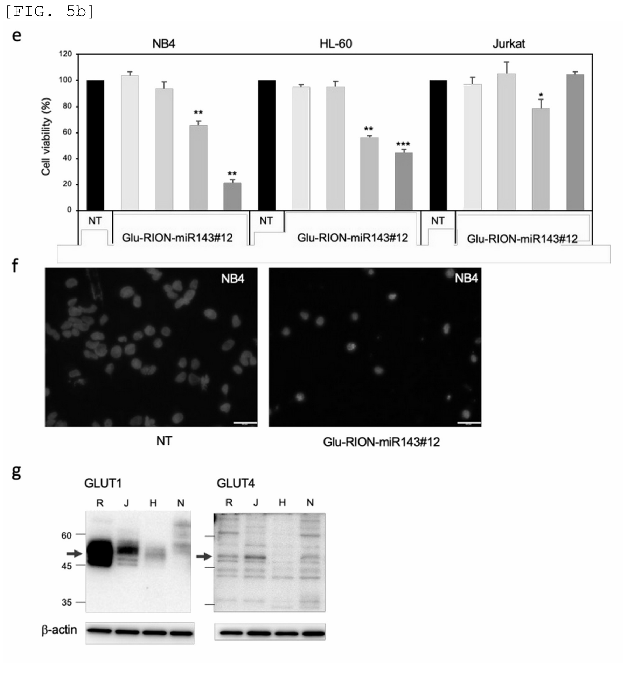

Fig. 5a is a diagram illustrating the effect of Glu-RION-miR143#12 on various hematologic malignancy cells. Various cells were treated for 72 hours, and the inhibitory effect on cell proliferation and other effects were examined (Figs. 5a, 5c, and 5e). The results illustrate fluorescence microscopic images of PRMI8226 cells treated with Glu-RION-miR143#12 and untreated PRMI8226 cells. Hoechst 33342 was used as a fluorescent reagent (Fig. 5d). In NB4 cells and HL-60 cells, even when the gene transfection reagent Lipofectamine was used instead of Glu-RION, no inhibitory effect on cell proliferation was observed (Fig. 5c).
Fig. 5b is a diagram illustrating the effect of Glu-RION-miR143#12 on various hematologic malignancy cells. Various cells were treated for 72 hours, and the inhibitory effect on cell proliferation and other effects were examined (Figs. 5a, 5c, and 5e). The results illustrate fluorescence microscopic images of PRMI8226 cells treated with Glu-RION-miR143#12 and untreated PRMI8226 cells. The presence of glucose transporters on hematologic malignancy cells was confirmed. NB4 cells were stained with Hoechst 33342 and then observed under a fluorescence microscope (Fig. 5f).
Fig. 6a is a diagram illustrating the results of Western blot analysis examining the expression of downstream factors in the RAS network in PRMI8226 cells treated with Glu-RION-miR143#12.
Fig. 6b is a diagram illustrating the results of Western blot analysis examining the expression of downstream factors in the RAS network in NB4, HL-60, and Jurkat cells treated with Glu-RION-miR143#12.
Fig. 7 is a diagram illustrating the relationship between the effect of Glu-RION-miR143#12 on the growth of human peripheral lymphocytes and the presence or absence of stimulation with concanavalin A.

### Description of Embodiments

Micelles or nanoparticles obtained from a composition containing a nucleic acid analog represented by the following formula (1) and miR-143 or an analog thereof, which are used in a therapeutic agent for treating hematologic malignancies of the present disclosure, will be described.

### (Hematologic malignancy)

The micelles or nanoparticles used in the present disclosure are used for treatment of hematologic malignancies. In the present disclosure, the term "hematologic malignancy" broadly refers to tumors classified into leukemia, malignant lymphoma, and multiple myeloma, and is generally considered to involve abnormalities in the K-RAS network in many cases. In many cases, these cancers are difficult to treat by surgical methods alone, unlike solid tumors. In the present disclosure, the expression "to treat hematologic malignancies" refers to inducing the death of, or inhibiting the proliferation of, tumor cells that collectively exist within the hematopoietic system or are present in a floating state in the blood or body fluids, by regulating the K-RAS network.

Leukemia is a general term for diseases in which white blood cells, or their immature precursor cells, proliferate uncontrollably. Leukemia is broadly classified into acute leukemia, chronic myelogenous leukemia, chronic lymphocytic leukemia, and adult T-cell leukemia. Acute leukemia is further classified into acute myelogenous leukemia and acute lymphocytic leukemia, and according to the French-American-British Classification (FAB classification), which is a diagnostic criterion that takes into account morphological findings, acute myelogenous leukemia can be further subclassified into acute promyelocytic leukemia, acute myelomonocytic leukemia, acute monocytic leukemia, acute erythroleukemia, and acute megakaryoblastic leukemia.

Malignant lymphoma is a disease in which "lymphocytes" in the blood become cancerous, and mainly occurs in lymphoid tissues such as the lymph nodes, spleen, and tonsils, but may also occur in organs other than lymphoid tissues, including the stomach, intestines, thyroid gland, lungs, liver, skin, bone marrow, and brain. Malignant lymphoma is pathologically classified into 50 or more types and is broadly divided into Hodgkin lymphoma and non-Hodgkin lymphoma. Non-Hodgkin lymphoma is classified into two types: B-cell type and T/NK-cell type, and is further divided into more detailed histopathological subtypes.

Multiple myeloma occurs when plasma cells, which are one type of blood cell, become cancerous. Plasma cells are cells differentiated from B lymphocytes, which are a type of white blood cell, and when plasma cells become cancerous and turn into abnormal cells (myeloma cells), the ability to attack foreign substances is lost. There are several types of plasma cell tumors, and in addition to multiple myeloma, plasmacytoma and macroglobulinemia are known.

(Nucleic acid to be delivered: miR-143 and analog thereof)

In the present disclosure, the nucleic acid to be delivered is a microRNA that regulates a network of the oncogene KRAS. MicroRNA (hereinafter, referred to as "miRNA") is an endogenous non-coding RNA of about 20 to 25 nucleotides that is encoded on the genome. miRNA is first transcribed from an miRNA gene on the genomic DNA as a primary transcript (PrimarymiRNA or Pri-miRNA) having a length of several hundred to several thousand nucleotides, and is then processed to form a precursor miRNA (pre-miRNA) having a hairpin structure of about 60 to 110 nucleotides. Thereafter, the precursor miRNA is transported from the nucleus into the cytoplasm and becomes a double-stranded miRNA of about 20 to 25 nucleotides through splicing. The double-stranded miRNA is incorporated into a protein called RISC, where it becomes a single-stranded miRNA (guide strand or antisense strand), while the other, less stable single strand (passenger strand or sense strand) is degraded. The single-stranded miRNA binds to the mRNA of a target gene having a partially complementary base sequence, thereby inhibiting translation of the target gene. Note that, in the present specification, "miR-143" may sometimes be referred to as "miR143".

1,000 or more types of miRNAs are known in humans, mice, and the like. Each miRNA regulates the expression of a plurality of target genes and is involved in various biological phenomena such as cell proliferation and differentiation. miRNAs have been suggested to be assembled with the onset and progression of diseases such as cancer, cardiovascular diseases, neurodegenerative diseases, psychiatric disorders, and chronic inflammatory diseases. In particular, it has been pointed out by many researchers that miRNAs are deeply involved in the proliferation of cancer cells, and research and development has been conducted on miRNAs as nucleic acid pharmaceuticals.

RAS forms an extensive network by regulating more than ten downstream signaling pathways. miR-143 is a suppressor miRNA that regulates the K-RAS network in a multifaceted manner and is capable of suppressing cancer growth. Many miR-143s that suppress cancer are known, and a number of miR-143 sequences (SEQ-1 to SEQ-23) have been proposed mainly by the inventors (illustrated in Tables 1 and 2, JP 6730717 B2). The analog of miR-143 as used in the present disclosure refers to a microRNA having a sequence and/or structure similar to those of the miR-143s described above and having an activity of regulating the K-RAS network in a multifaceted manner.

**[Table 1]**

| | Strand name | | 5'-end modification | Oligonucleotide (5' → 3') | 3'-end modification |
|---|---|---|---|---|---|
| **SEQ-1** | Sense strand | **S-1** | | U*G*A*G*G*U*G*C*A*G*U*G*C*U*G*C*A* U*C*U*C*U*G*G | |
| | Antisense strand | **AS-4** | | Uf^Gm^Af*Gm*Af*Um*Gf*Am*Af*Gm*Cf*Am *Cf*Um*Gf*Um*Af*Gm*Cf*Um*Cf*Am | **^dT^dT** |
| **SEQ-2** | Sense strand | **S-1** | | U*G*A*G*G*U*G*C*A*G*U*G*C*U*G*C*A* U*C*U*C*U*G*G | |
| | Antisense strand | **AS-7** | **-P(O)(OH)₂** | Uf^Gm^Af*Gm*Af*Um*Gf*Am*Af*Gm*Cf*Am *Cf*Um*Gf*Um*Af*Gm*Cf*Um*Cf*Am | **^dT^dT** |
| **SEQ-3** | Sense strand | **S-17** | | Uf^G^A*G*G*Uf*G*Cf*A*G*Uf*G*Cf*Uf*G*Cf *A*Uf*Cf*Uf*Cf*Uf*G*G | |
| | Antisense strand | **AS-4** | | Uf^Gm^Af*Gm*Af*Um*Gf*Am*Af*Gm*Cf*Am *Cf*Um*Gf*Um*Af*Gm*Cf*Um*Cf*Am | |
| **SEQ-4** | Sense strand | **S-1** | | U*G*A*G*G*U*G*C*A*G*U*G*C*U*G*C*A* U*C*U*C*U*G*G | |
| | Antisense strand | **AS-30** | | Um^Gm^Am*G*A*U*Gm*Am*Am*G*C*A*Cm*Um* Gm*U*A*G*Cm*Um*Cm*Am | **^dT^dT** |
| **SEQ-5** | Sense strand | **S-1** | | U*G*A*G*G*U*G*C*A*G*U*G*C*U*G*C*A* U*C*U*C*U*G*G | |
| | Antisense strand | **AS-31** | | Uf^Gf^Af*Gm*Am*Um*Gf*Af*Af*Gm*Cm* Am*Cf*Uf*Gf*Um*Am*Gm*Cf*Uf*Cf*Af | **^dT^dT** |
| **SEQ-6** | Sense strand | **S-17** | | Uf^G^A*G*G*Uf*G*Cf*A*G*Uf*G*Cf*Uf*G*Cf *A*Uf*Cf*Uf*Cf*Uf*G*G | |
| | Antisense strand | **AS-3** | | Uf^G^A*G*A*Uf*G*A*A*G*Cf*A*Cf*Uf* G*Uf*A*G*Cf*Uf*Cf*A | **^dT^dT** |
| **SEQ-7** | Sense strand | **S-17** | | Uf^G^A*G*G*Uf*G*Cf*A*G*Uf*G*Cf*Uf*G*Cf *A*Uf*Cf*Uf*Cf*Uf*G*G | |
| | Antisense strand | **AS-10** | | Uf^Gm^Af*Gm*Af*Um*Gf*Am*Af*Gm*Cf*Am *Cf*Um*Gf*Um*Af*Gm*Cf*Um*Cf*Am | **^dG^dG** |
| **SEQ-8** | Sense strand | **S-18** | | Um^G^A*G*G*Um*G*Cm*A*G*Um*G*Cm*Um* G*Cm*A*Um*Cm*Um*Cm*Um*G*G | |
| | Antisense strand | **AS-12** | | Uf^Gm^Af*Gm*Af*Um*Gf*Am*Af*Gm*Cf*Am*Cf*Um *Gf*Um*Af*Gm*Cf*Um*Cf*Am | **^Gₘ^Gₘ** |
| **SEQ-9** | Sense strand | **S-18** | | Um^G^A*G*G*Um*G*Cm*A*G*Um*G*Cm*Um* G*Cm*A*Um*Cm*Um*Cm*Um*G*G | |
| | Antisense strand | **AS-13** | | UfGm^AfGm*AfUm*GfAm*AfGm*CfAm*CfUm *GfUm*AfGm*CfUm*CfAm | **^Gr^Gr** |
| **SEQ-10** | Sense strand | **S-19** | | Uf^G^A*G*G*Uf*G*C*A*G*Uf*G*C*Uf*G*C* A*Uf*C*Uf*C*Uf*G*G | |
| | Antisense strand | **AS-3** | | Uf^G^A*G*A*Uf*G*A*A*G*Cf*A*Cf*Uf* G*Uf*A*G*Cf*Uf*Cf*A | **^dT^dT** |
| **SEQ-11** | Sense strand | **S-1** | | U*G*A*G*G*U*G*C*A*G*U*G*C*U*G*C*A* U*C*U*C*U*G*G | |
| | Antisense strand | **AS-42** | **VP-** | Um^Gm^Af*Gm*Af*Um*Gf*Am*Af*Gm*Cf* Am*Cf*Um*Gf*Um*Af*Gm*Cf*Um*Cf*Am | **^dT^dT** |
| **SEQ-12** | Sense strand | S-1 | | U*G*A*G*G*U*G*C*A*G*U*G*C*U*G*C*A* U*C*U*C*U*G*G | |
| | Antisense strand | **AS-47** | | Uf^Gm^Af*Gm*Af*Um*Gf*Am*Af*Gm*Cf*Am* Cf*Um*Gf*Um*Af*Gm*Cf*Um*Ab*Ab | **^dT^dT** |

**[Table 2]**

| | Strand name | | 5'-end modification | Oligonucleotide (5' → 3') | 3'-end modification |
|---|---|---|---|---|---|
| **SEQ-13** | Sense strand | **S-18** | | Um^G^A*G*G*Um*G*Cm*A*G*Um*G*Cm*Um* G*Cm*A*Um*Cm*Um*Cm*Um*G*G | |
| | Antisense strand | **AS-47** | | Uf^Gm^Af*Gm*Af*Um*Gf*Am*Af*Gm*Cf*Am* Cf*Um*Gf*Um*Af*Gm*Cf*Um*Ab*Ab | **^dT^dT** |
| **SEQ-14** | Sense strand | **S-1** | | U*G*A*G*G*U*G*C*A*G*U*G*C*U*G*C*A* U*C*U*C*U*G*G | |
| | Antisense strand | **AS-50** | **-P(O)(OH)₂** | Uf^Gm^Af*Gm*Af*Um*Gf*Am*Af*Gm*Cf*Am* Cf*Um*Gf*Um*Af*Gm*Cf*Um*Cf*Am | **^dG^dG** |
| **SEQ-15** | Sense strand | **S-1** | | U*G*A*G*G*U*G*C*A*G*U*G*C*U*G*C*A* U*C*U*C*U*G*G | |
| | Antisense strand | **AS-51** | **-P(O)(OH)₂** | Uf^Gm^Af*Gm*Af*Um*Gf*Am*Af*Gm*Cf*Am* Cf*Um*Gf*Um*Af*Gm*Cf*Um*Cf*Am | **^Gₘ^Gₘ** |
| **SEQ-16** | Sense strand | **S-1** | | U*G*A*G*G*U*G*C*A*G*U*G*C*U*G*C*A* U*C*U*C*U*G*G | |
| | Antisense strand | **AS-52** | **-P(O)(OH)₂** | Uf^Gm^Af*Gm*Af*Um*Gf*Am*Af*Gm*Cf*Am* Cf*Um*Gf*Um*Af*Gm*Cf*Um*Cf*Am | **^Gr^Gr** |
| **SEQ-17** | Sense strand | **S-1** | | U*G*A*G*G*U*G*C*A*G*U*G*C*U*G*C*A* U*C*U*C*U*G*G | |
| | Antisense strand | **AS-55** | **-P(O)(OH)₂** | Uf^Gf^Af*Gm*Am*Um*Gf*Af*Af*Gm*Cm*Am* Cf*Uf*Gf*Um*Am*Gm*Cf*Uf*Cf*Af | **^dT^dT** |
| **SEQ-18** | Sense strand | **S-1** | | U*G*A*G*G*U*G*C*A*G*U*G*C*U*G*C*A* U*C*U*C*U*G*G | |
| | Antisense strand | **AS-56** | **-P(O)(OH)₂** | Uf^Gm^Af*Gm*Af*Um*Gf*Am*Af*Gm*Cf*Am* Cf*Um*Gf*Um*Af*Gm*Cf*Um*Ab*Ab | **^dT^dT** |
| **SEQ-19** | Sense strand | **S-17** | | Uf^G^A*G*G*Uf*G*Cf*A*G*Uf*G*Cf*Uf*G*Cf *A*Uf*Cf*Uf*Cf*Uf*G*G | |
| | Antisense strand | **AS-56** | **-P(O)(OH)₂** | Uf^Gm^Af*Gm*Af*Um*Gf*Am*Af*Gm*Cf*Am* Cf*Um*Gf*Um*Af*Gm*Cf*Um*Ab*Ab | **^dT^dT** |
| **SEQ-20** | Sense strand | **S-18** | | U~m~^G^A*G*G*U~m~*G*C~m~*A*G*U~m~*G*C~m~*U~m~* G*C~m~*A*U~m~*C~m~*U~m~*C~m~*U~m~*G*G | |
| | Antisense strand | **AS-56** | **-P(O)(OH)₂** | Uf^Gm^Af*Gm*Af*Um*Gf*Am*Af*Gm*Cf*Am* Cf*Um*Gf*Um*Af*Gm*Cf*Um*Ab*Ab | **^dT^dT** |
| **SEQ-21** | Sense strand | **S-1** | | U*G*A*G*G*U*G*C*A*G*U*G*C*U*G*C*A* U*C*U*C*U*G*G | |
| | Antisense strand | **AS-57** | **-P(O)(OH)₂** | Um^Gf^Am*Gf^Am*Uf^Gm*Af^Am*Gf^Cm*Af^ Cm*Uf^Gm*Uf^Am^Gf^Cm^Uf^Cm^Af | **^Aₘ^Aₘ** |
| **SEQ-22** | Sense strand | **S-17** | | Uf^G^A*G*G*Uf*G*Cf*A*G*Uf*G*Cf*Uf*G*Cf *A*Uf*Cf*Uf*Cf*Uf*G*G | |
| | Antisense strand | **AS-57** | **-P(O)(OH)₂** | Um^Gf^Am*Gf^Am*Uf^Gm*Af^Am*Gf^Cm*Af^ Cm*Uf^Gm*Uf^Am^Gf^Cm^Uf^Cm^Af | **^Aₘ^Aₘ** |
| **SEQ-23** | Sense strand | **S-18** | | Um^G^A*G*G*Um*G*Cm*A*G*Um*G*Cm*Um* G*Cm*A*Um*Cm*Um*Cm*Um*G*G | |
| | Antisense strand | **AS-57** | **-P(O)(OH)₂** | Um^Gf^Am*Gf^Am*Uf^Gm*Af^Am*Gf^Cm*Af^ Cm*Uf^Gm*Uf^Am^Gf^Cm^Uf^Cm^Af | **^Aₘ^Aₘ** |

In the sequences, A, U, G, and C each represent RNA containing adenine, uracil, guanine, or cytosine, respectively; dT and dG each represent DNA containing thymine or guanine, respectively; RNAf represents 2'-FRNA, RNAm represents 2'-OMeRNA, ^ represents -P(S)OH-, * represents -P(O)OH-, and Ab(Abasic) represents a group illustrated below:

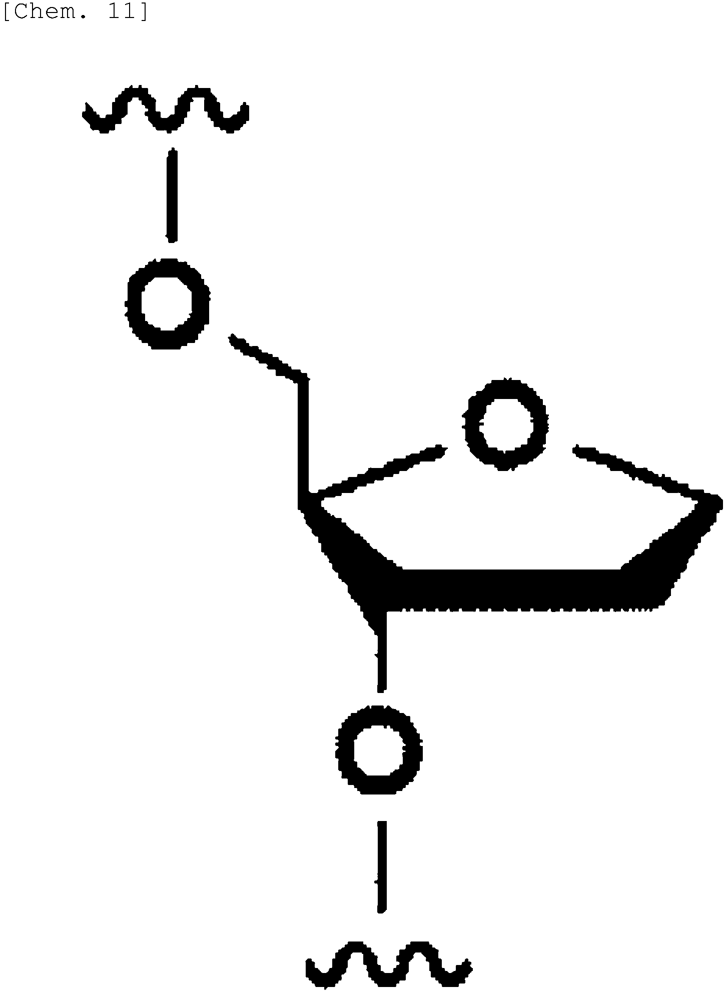

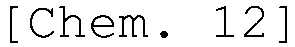

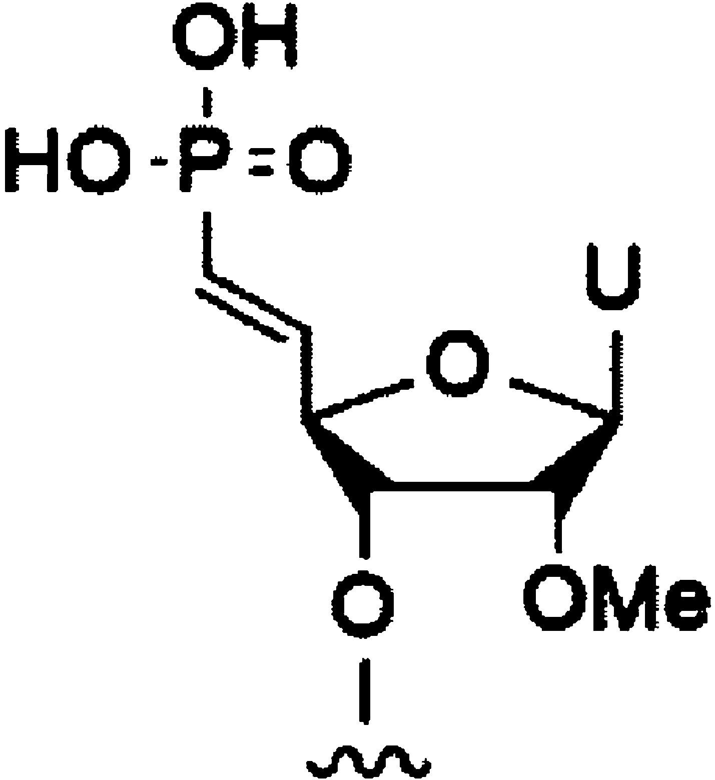

VP- at the 5' end of AS-42 and Um at the end of the oligonucleotide constitute the groups illustrated below, in which the formula: =CH-P(=O)(OH)₂ corresponds to the 5'-end modification (VP-).

Note that the sequences and sequence numbers in the above tables in the sequence listing are as follows.

S-1 (SEQ ID NO: 1), S-17 (SEQ ID NO: 2), S-18 (SEQ ID NO: 3), S-19 (SEQ ID NO: 4), AS-4 (SEQ ID NO: 5), AS-7 (SEQ ID NO: 6), AS-30 (SEQ ID NO: 7), AS-31 (SEQ ID NO: 8), AS-3 (SEQ ID NO: 9), AS-10 (SEQ ID NO: 10), AS-12 (SEQ ID NO: 11), AS-13 (SEQ ID NO: 12), AS-42 (SEQ ID NO: 13), AS-47 (SEQ ID NO: 14), AS-50 (SEQ ID NO: 15), AS-51 (SEQ ID NO: 16), AS-52 (SEQ ID NO: 17), AS-55 (SEQ ID NO: 18), AS-56 (SEQ ID NO: 19), and AS-57 (SEQ ID NO: 20)

Among the above, SEQ-8 (sense strand S-18 and antisense strand AS-12) illustrated in the above table is preferred for hematologic malignancies, which are the target of the present disclosure.

### (Nucleic acid analog)

Hereinafter, the nucleic acid analog of the present disclosure is described. The nucleic acid analog of the present disclosure includes a cationic artificial nucleic acid and a hydrophilic polymer bound to the cationic artificial nucleic acid. The nucleic acid analog is preferably used as a carrier for delivering a nucleic acid that is to be delivered (hereinafter, may be referred to as a "nucleic acid to be delivered") to a target site. The nucleic acid to be delivered of the present disclosure is miR-143 or a derivative of the miR-143. The nucleic acid analog of the present disclosure can be represented by the following formula (1):

[Chem. 13] **L-(S2)s-H- (S1)t-N (1**)

wherein N represents a cationic artificial nucleic acid, H represents a hydrophilic polymer, S1 represents a spacer 1, S2 represents a spacer 2, L represents a ligand, s represents 0 or 1, and t represents 0 or 1.

### (Cationic artificial nucleic acid)

Among the elements constituting the nucleic acid analog, the cationic artificial nucleic acid represented by N has structural units in which a base is bonded to a hexose selected from ribose and deoxyribose, linking structures linking two of the structural units, and a backbone structure having cationic groups. The base sequence of the cationic artificial nucleic acid can be appropriately designed in accordance with the base sequence of the nucleic acid to be delivered, taking into consideration base complementarity, electrostatic interaction strength, and the like.

The cationic group, in a cationic state, has a partial structure selected from the group consisting of the following formulas (C1) to (C7): 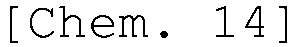 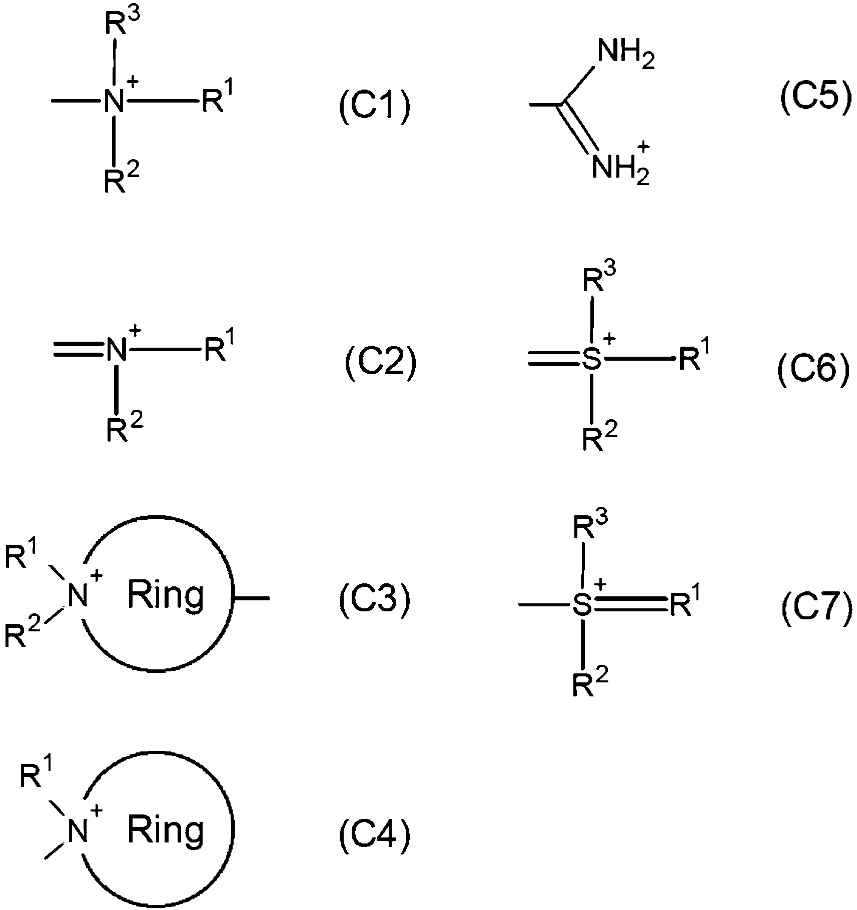 wherein R¹ to R³ represent hydrogen or an alkyl group having 1 to 10 carbon atoms, R⁶ and R⁷ may be the same as or different from each other, and Ring is a cyclic compound including 4 to 8 carbon atoms, and may be a heterocyclic ring in which one or more of the carbon atoms are substituted with a heteroatom selected from nitrogen, oxygen, and sulfur.

The cationic artificial nucleic acid can assemble through electrostatic interaction between a phosphate group of another nucleotide and the cationic group. Here, the "another nucleotide" means a nucleotide such as DNA or RNA or an analog thereof, and when a nucleic acid analog is used as a carrier, it means a nucleotide constituting a nucleic acid to be delivered or an analog thereof.

The pKa of the cationic group is higher than the pKa of the phosphate group of another nucleotide from the viewpoint of electrostatic interaction with the phosphate group of another nucleotide. Under a pH condition lower than the pKa of the cationic group of the nucleic acid analog and higher than the pKa of the phosphate group of another nucleotide, the cationic group is positively charged and the phosphate group is negatively charged, so that these groups are electrostatically bonded. Here, in general, since the pKa of the phosphate group of the nucleotide is less than 1, the pKa of the cationic group is 1 or more, preferably 3 or more, and more preferably 6.0 or more. The upper limit of the pKa of the cationic group is not particularly limited, but is 12 or less, preferably 11 or less, and more preferably 9 or less. The pKa of the cationic group is preferably within the range of 6 to 9 from the viewpoint of the strength of electrostatic interaction with the phosphate group of another nucleotide, the structure of the cationic artificial nucleic acid, and the like.

Examples of the base include adenine, guanine, cytosine, thymine, uracil, N-methyladenine, N-benzoyladenine, 2-methylthioadenine, 2-aminoadenine, 7-methylguanine, N-isobutyrylguanine, 5-fluorocytosine, 5-bromocytosine, 5-methylcytosine, 4-N-methylcytosine, 4-N,N-dimethylcytosine, 5-fluorouracil, 5-bromouracil, 5-chlorouracil, and 5,6-dihydrouracil.

The linking structure, in a cationic state, is preferably one having at least a structure selected from the following formulas (L1) to (L4): 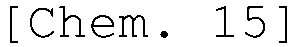 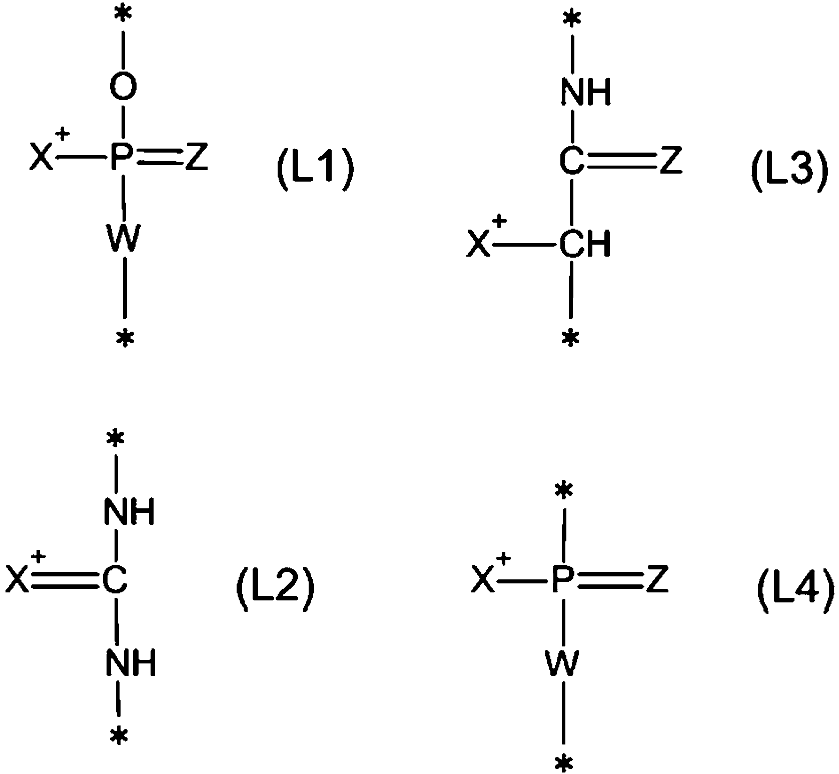 wherein X⁺ is a functional group containing the cationic group of the formulas (C1) to (C7), Z represents O or S, W represents -O- or -NR⁴-, wherein R⁴ represents hydrogen or an alkyl group having 1 to 10 carbon atoms, and * denotes a bond with the structural unit adjacent thereto.

Here, the phrase "in a cationic state" means a case wherein the cationic group is assumed to be positively charged, and there is a case wherein the cationic group is not positively charged depending on environmental conditions (pH, type, and the like of the solvent) in which the nucleic acid analog is placed. When the cationic group is ammonium taking the above formula (C1) as an example, examples of the state of not being positively charged include states of a primary amine (primary ammonium when cationized), a secondary amine (secondary ammonium when cationized), a tertiary amine (tertiary ammonium when cationized), and the like.

Examples of the cationic artificial nucleic acid include those having a nucleotide backbone having, as a structural unit, a nucleotide in which a base is bonded to ribose or deoxyribose. Note that the cationic artificial nucleic acid also includes those having a morpholino backbone having, as a structural unit, a structure in which a base is bonded to morpholine. 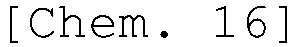 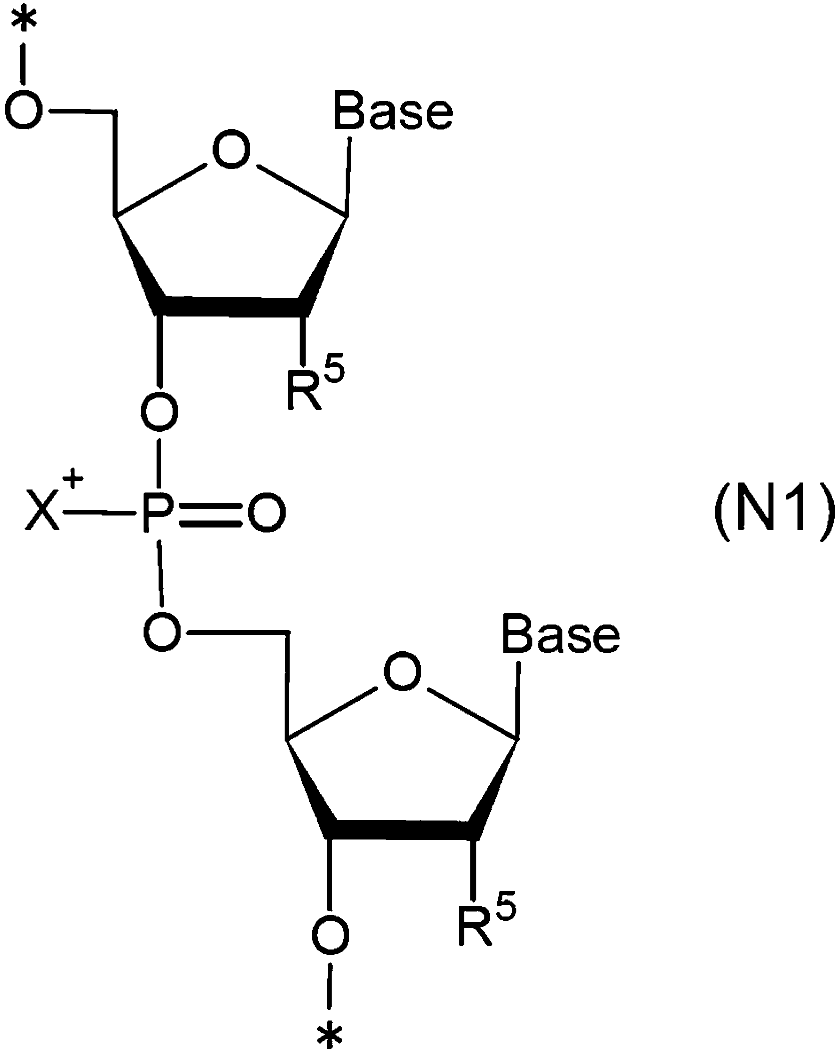 Wherein Base represents a base, R⁵ represents H or OH, * denotes a bond with a phosphate of an adjacent nucleotide backbone, at least one of * is bonded to the hydrophilic polymer at the 5' end or 3' end, and when not bonded to the hydrophilic polymer, * is hydrogen.

Examples of the structure having a nucleotide backbone include the following.

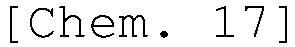

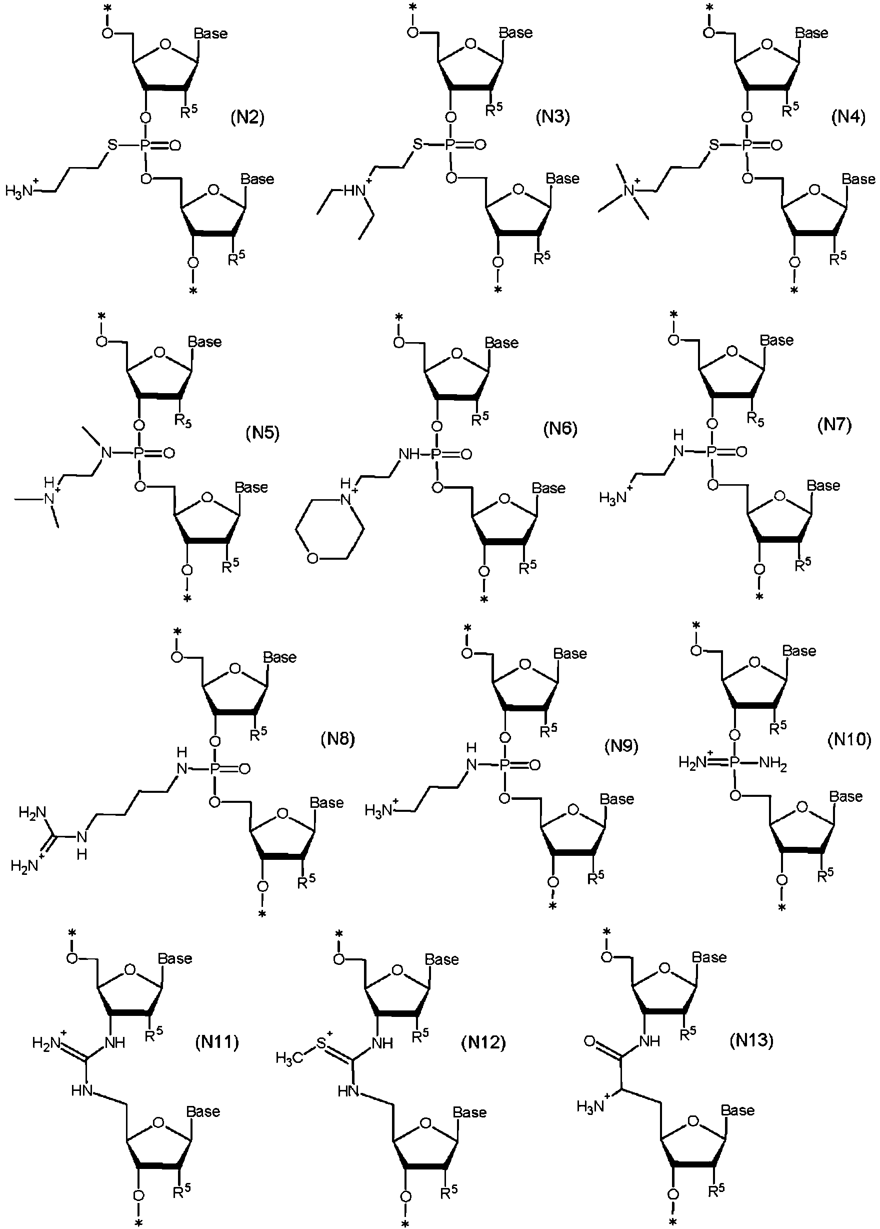

In the above case, X⁺, in a cationic state, is preferably an ammonium cation represented by the following formula (F1): 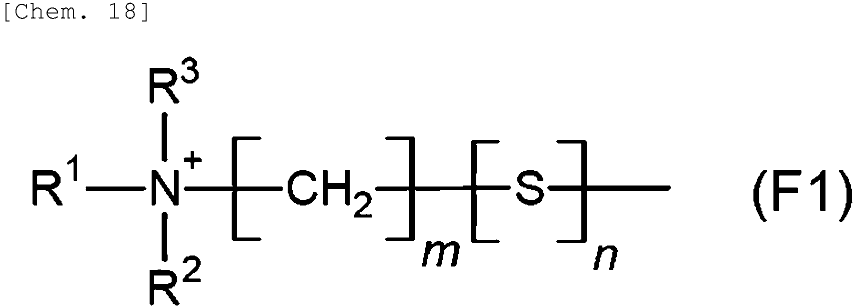 wherein R¹ to R³ represent hydrogen or an alkyl group having 1 to 10 carbon atoms, and may be the same as or different from each other, m represents an integer between 0 and 10, and n represents an integer of 0 or 1.

Here, the strength of electrostatic interaction with the phosphate group of another nucleotide is primary amine (R¹ to R³ are all hydrogen) < secondary amine (two of R¹ to R³ are hydrogen, and the other is an alkyl group) < tertiary amine (one of R¹ to R³ is hydrogen, and the others are alkyl groups) < quaternary ammonium (R¹ to R³ are all alkyl groups). In addition, as illustrated in Examples described below, a construct having quaternary ammonium as a cationic group has a property of being likely to collapse as the pH of the environment decreases, as compared with one having tertiary amine and the like. This means that if a construct having quaternary ammonium as a cationic group is transferred to cytoplasm via endosome, the construct is likely to collapse in response to a weakly acidic endosomal pH and release a nucleic acid to be delivered. Therefore, among these, the cationic group of X⁺ is particularly preferably quaternary ammonium. The pKa of the cationic group is about 6.1 to 7.9 in the case of primary amine, 6.9 to 7.0 in the case of secondary amine, 8.0 to 8.6 in the case of tertiary amine, and about 8.0 to 9.0 in the case of quaternary ammonium, depending on the structure.

On the other hand, examples of the structure having a morpholino backbone include a structure represented by the following formula (M1): 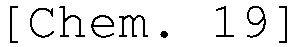 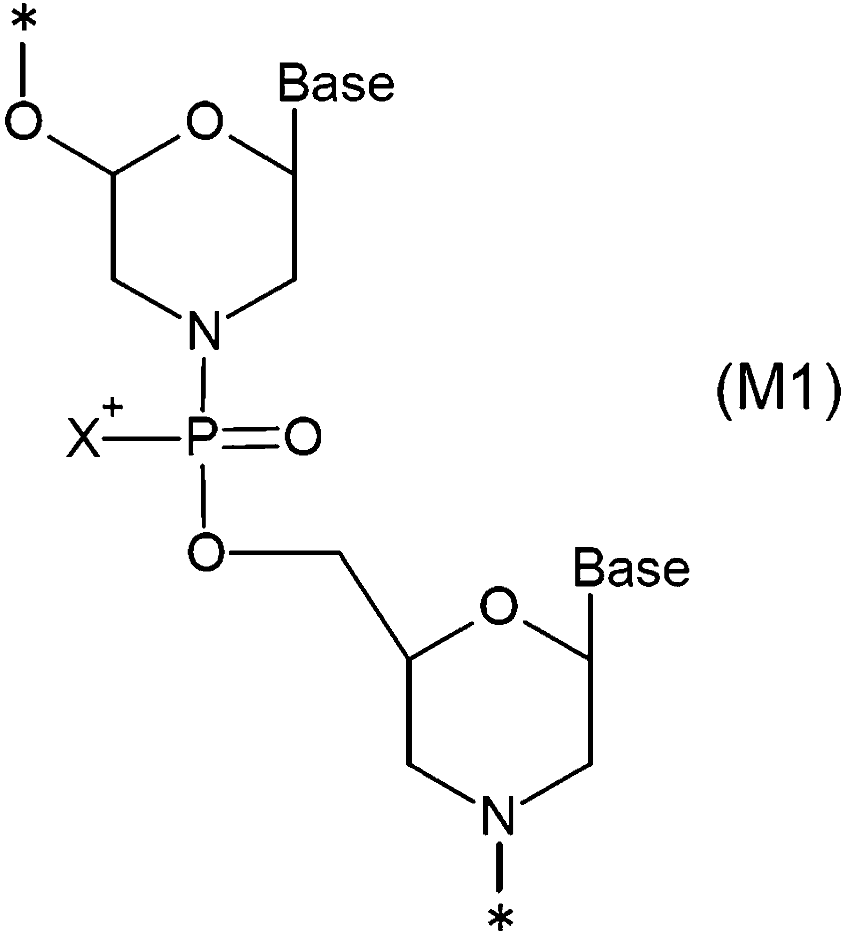 wherein Base represents a base, * denotes a bond with phosphorus of an adjacent morpholino backbone, at least one of * is bonded to the hydrophilic polymer at the 5' end or 3' end, and when not bonded to the hydrophilic polymer, * is hydrogen.

Examples of the structure having a morpholino backbone include the following.

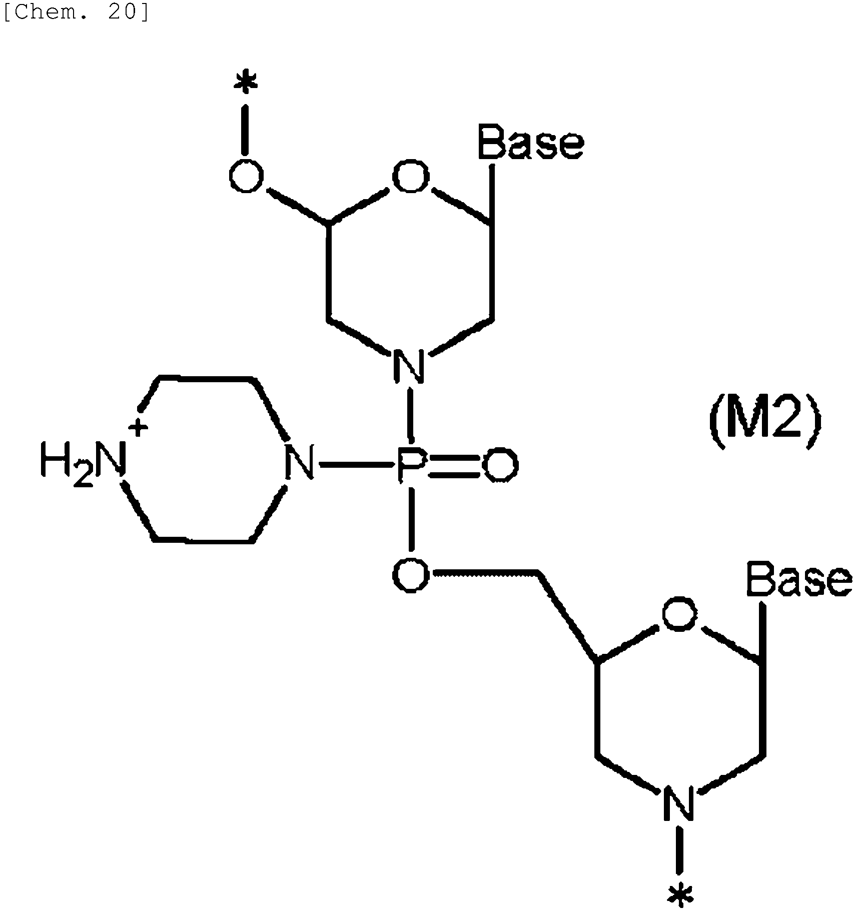

The number of structural units in which a ring structure and a base constituting the cationic artificial nucleic acid are bonded to each other (degree of polymerization) can be appropriately determined according to conditions such as the type of the ring structure and the cationic group, and the type and length (number of bases) of the nucleic acid to be delivered. In general, the degree of polymerization of the cationic artificial nucleic acid is about 5 to 100, preferably about 10 to 50. When the degree of polymerization (number of bases) of the nucleic acid to be delivered is as short as about 20 bases, it is preferable that the degree of polymerization of the cationic artificial nucleic acid as a carrier is also about the same as that of the nucleic acid to be delivered. On the other hand, when a relatively long nucleic acid such as mRNA is assumed to be delivered, the ratio of the negative charge to the length of the nucleic acid is smaller, and the influence of a portion having a negative charge on the entire nucleic acid is smaller. Therefore, the degree of polymerization of the cationic artificial nucleic acid may be different from the degree of polymerization of the nucleic acid to be delivered as long as the assembly between the cationic artificial nucleic acid and the nucleic acid to be delivered is not hindered. For example, when the nucleic acid to be delivered is a long nucleic acid such as mRNA, the degree of polymerization of the cationic artificial nucleic acid may be smaller than the degree of polymerization of the nucleic acid to be delivered.

In the cationic artificial nucleic acid, the cationic group is introduced into a part or whole of a linking structure that bonds structural units, but from the viewpoint of structure formation of a vesicle and a micelle to be described below and the like, the ratio of the linking structure into which the cationic group is introduced is preferably 50% or more, more preferably 80% or more, and particularly preferably 100% (whole linking structure) with respect to the total number of linking structures. When the ratio of the number of linking structures into which a cationic group is introduced to the total number of linking structures is low, in the assembly structure formed between the cationic artificial nucleic acid and the nucleic acid to be delivered, the negative charge of the nucleic acid to be delivered becomes dominant, and a construct is hardly formed. Alternatively, when a cationic group is sequentially introduced into one of the 3' and 5' ends, or when a cationic group is introduced into an intermittent linking structure such as an alternate linking structure, it is conceivable to form a construct, but it is considered that the pH response of these structures changes depending on the introduction ratio of cations, and the structure is unstable. Therefore, the ratio of the linking structure into which the cationic group is introduced is preferably high.

### (Hydrophilic polymer)

In the formula (1), H represents a hydrophilic polymer.

As the hydrophilic polymer, various polymers can be used depending on the use of the nucleic acid analog and the like. In particular, when a nucleic acid analog is used as a carrier for delivering a nucleic acid to a target site, the hydrophilic polymer preferably has biocompatibility. In addition, since the nucleic acid analog is cationic, the hydrophilic polymer is preferably a neutral polymer which hardly electrically interacts (attraction, repulsion) with the nucleic acid analog.

Such a hydrophilic polymer is preferably selected from polyethylene glycol, polyvinyl alcohol, polyglutamic acid, polyvinylpyrrolidone, polyacrylamide, polyethyleneimine, polyalkyl acrylate, polyoxazoline, polyacrylamide, poly(carboxybetaine methacrylate), poly(sulfobetaine methacrylate), poly(2-methacryloyloxyethyl phosphocholine), hyaluronic acid, chitosan, dextran, and derivatives thereof.

Examples of the hydrophilic polymer include the following.

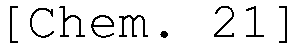

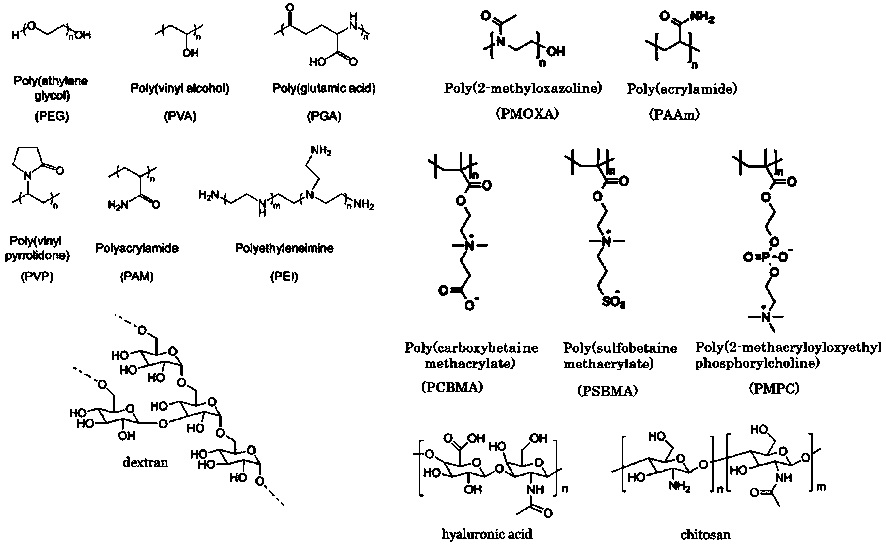

The type of monomer constituting the hydrophilic polymer and the number of monomer units (degree of polymerization) can be appropriately determined depending on conditions such as the type and molecular weight of the nucleic acid to be delivered, the molecular weight of the artificial nucleic acid, and the surrounding environment of the target site to which the nucleic acid is delivered. Representative examples of the type of monomer include oligomers of ethylene glycol, propylene glycol, and butylene glycol units, and the degree of polymerization of the hydrophilic polymer is generally about 2 to 100, preferably about 2 to 50, and more preferably about 2 to 10. In addition, the oligomers of these hydrophilic polymers may also be repeated two or more times via a phosphodiester group or the like. The number of repeats via a phosphodiester bond may be 3 or more, and preferably 4 or more, and the number of repeats of the hydrophilic polymer is at most 100 or less, preferably 30 or less, and more preferably 10 or less, depending on the balance with the molecular weight of the artificial nucleic acid, complementarity with the nucleic acid to be delivered, and hydrophobicity upon assembly through electrostatic interaction. When the degree of polymerization or the number of repeats of the hydrophilic polymer is low, it becomes difficult to form the construct described below, and the resulting construct tends to have low degradation stability and blood retention because it is likely to be eliminated as a foreign substance in vivo. Conversely, when the degree of polymerization of the hydrophilic polymer is too high, the size of the construct is too large, and the delivery efficiency of the nucleic acid to be delivered to the target site tends to be low.

In particular, when a nucleic acid analog is used as a carrier of a drug delivery system (DDS), polyethylene glycol is particularly preferable as the hydrophilic polymer from the viewpoint of blood retention and the like. As the polyethylene glycol, a 10 to 100 K polyethylene glycol may be used, but it is preferable to have a polyethylene glycol backbone represented by the following formula (A1): 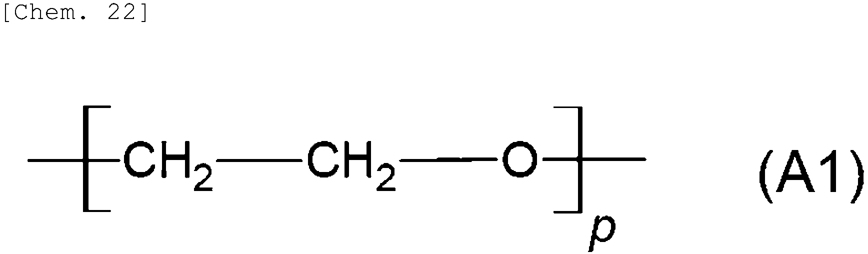 wherein p represents an integer between 1 and 20.

A1 can also be used as a polymer having a nucleobase or a phosphate ester derivative (-PO(OH)-, - PS(OH)-, PO(SH)-) as a monomer unit. The number of repeats of ethylene glycol of the monomer unit A1 (q) is 1 to 20, preferably 3 to 10, the structure of the monomer may be the same or different, and the degree of polymerization of the monomer unit is 1 to 10, preferably 2 to 10, and more preferably 2 to 8. Examples of such a phosphate ester derivative monomer unit include an ethylene glycol phosphate ester derivative unit, a diethylene glycol phosphate ester derivative unit, a triethylene glycol phosphate ester derivative unit, and a hexaethylene glycol phosphate ester derivative unit.

These derivative units can also be used as monomers of a polymer. The degree of polymerization of the phosphate ester derivative unit is 1 to 10, preferably 2 to 10, and more preferably 2 to 8, when the number of bases of the artificial nucleic acid is 10 to 30, depending on the size of the artificial nucleic acid. It is most preferable that the monomer unit is a triethylene glycol phosphate ester derivative or a hexaethylene glycol phosphate ester derivative. The type and degree of polymerization of the monomer of the hydrophilic polymer described above, particularly the degree of polymerization of the ethylene glycol phosphate derivative monomer, can be selected from the viewpoints of easiness of formation of a construct to be described below, uneasiness of recognition as a foreign substance in a living body, lowering of decomposition stability and blood retention, and the like.

### (Ligand)

The surface of the construct may be modified with various ligands. By modifying the surface of the construct with such a ligand, target directivity or the like can be imparted to the construct. Examples of the ligand include immunoglobulins, carbohydrates, peptides, proteins, and aptamers. The main ligands and their application fields predicted from studies on lipid nanoparticles are as follows. In the present disclosure, in spite of the following expectations, it was demonstrated that when glucose was used as a ligand, it was also effective against hematologic malignancies of suspension-type cells.

· Glucose: tumor study, drug delivery to brain capillary endothelial cells
·Mannose: efficient formation of large liposomes
·Galactose: study of galactose receptors in macrophages, study of targeted delivery of galactose to hepatocytes
·Sucrose: cancer treatment with doxorubicin
·Maltose: transport of doxorubicin in cancer treatment
· Lactose: studies of liposome size and stability
·Oligosaccharides: design of therapeutic inhibitors
· Lectin: pulmonary drug delivery
·Tomato lectin, wheat germ agglutinin: oral administration of insulin
· NCL-Aptamer: chemotherapy using cisplatin against wide range of cancers
·sgc8 Aptamer: for leukemia CEM-CCRF cells
·NX 1838: specific binding to VEGF of cancer cells
·Anti-CD44: selective targeting of cancer cells
·DAG-NX213: specificity to VEGF for promoting angiogenesis
· AS1411: cytotoxicity against breast cancer cells MCF-7
· Macugen: therapeutic agent for macular age-related macular degeneration
· BOCK: use for recognizing different binding sites of thrombin
· TASSET: use for recognizing different binding sites of target proteins
· xPSM-A9: use against prostate-specific membrane antigen expressed on prostate cancer cells
· IL-4Rα: inhibition of tumor growth utilizing tumor microenvironment

### (Spacer 1)

The bond between the cationic artificial nucleic acid and the hydrophilic polymer may be bonded in various bonding modes, or may be bonded via a linker. Examples of such a bond include an ester bond (-C(=O)-O-), an ether bond (-O-), a disulfide bond (-S-S-), phosphoramide (-P(OH)-NH-), and phosphate ester (-OPO-O-). As the linker, these may be used alone or in combination, and an ester bond, an ether bond, a disulfide bond, or the like of an alkyl chain may be interposed therebetween. When the cationic artificial nucleic acid has a nucleotide backbone, it is preferable that these linkers are bonded to a hydroxyl group at the 5' end, the 3' end, or both of the cationic artificial nucleic acid, and are bonded so as to connect the cationic artificial nucleic acid and the hydrophilic polymer via the linkers. When the cationic artificial nucleic acid and the hydrophilic polymer are bonded to each other by a linker, the linker is different in structure from the hydrophilic polymer, so that it can be expected that the charge is regulated. In addition, in the case of interposing a phosphoramidite, it is preferable because it behaves as a negative charge similarly to a polymer made of polyethylene glycol. Further, it is also advantageous in that the induction of PEG antibody is suppressed. Furthermore, in the case of interposing a phosphoramidite, it is industrially advantageous in that the nucleic acid sequence to the hydrophilic polymer portion can be consistently synthesized by automatic nucleic acid synthesis.

The spacer 1 is a structure that links a cationic artificial nucleic acid and a hydrophilic polymer. The spacer 1 (S1) may have a bond represented by the following formula (S11):

[Chem. 23] **-O-R⁶-S-S-R⁷-O- (S11)**

wherein R₆ and R₇ in the formula represent a methylene group having 1 to 12 carbon atoms, and R₆ and R₇ may be the same as or different from each other.

### (Spacer 2)

When the ligand is an aldose and the corresponding phosphoramidite reagent is unstable, the spacer 22 can be used. The spacer 22 is a structure that links a hydrophilic polymer and a ligand. It is preferable that the spacer 2 (S2) is a phosphodiester bond, or a phosphodiester bond including a triazole illustrated in the formula (S21).

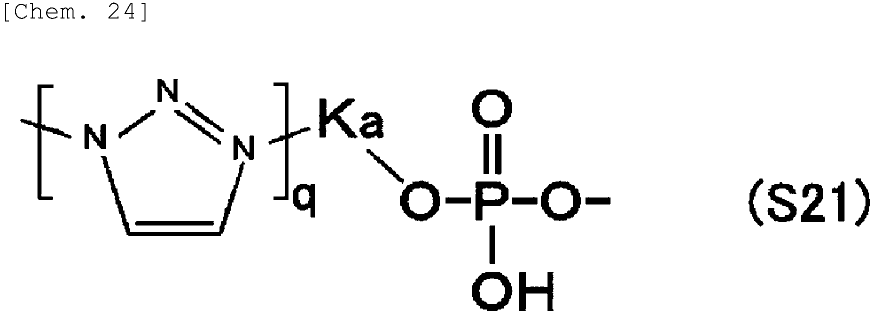

In the formula, Ka represents an amide bond containing a methylene group having 1 to 20 carbon atoms, a compound containing an aromatic ring having 6 to 12 carbon atoms, or a direct bond, q is 0 or 1, and when q is 0, the 1,2,3-triazole ring is bound to the ligand. The spacer 1 and the spacer 2 are optional structures in the nucleic acid analog and may be provided as necessary.

### (Nucleic acid delivery construct)

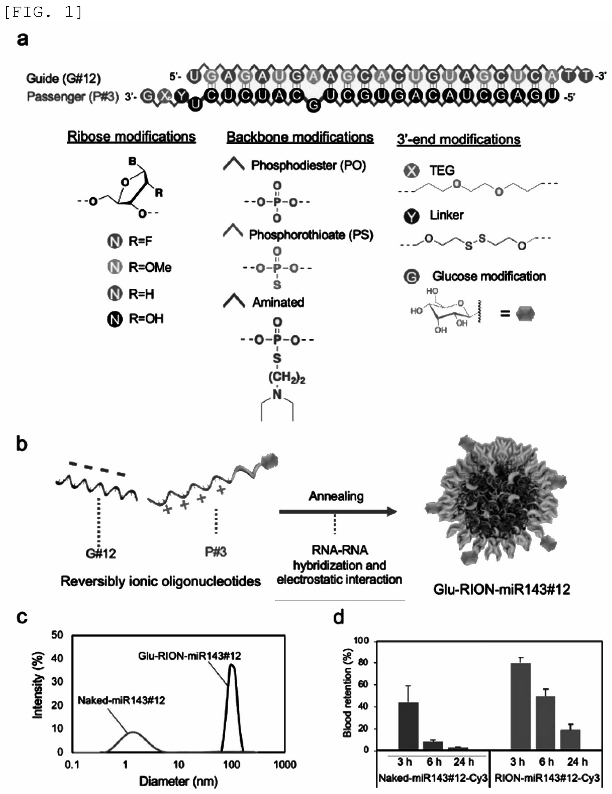

The nucleic acid analog of the present disclosure can be particularly preferably used as a carrier for delivering a nucleic acid to be delivered to a target site. Fig. 1 is a schematic diagram illustrating a case where a nucleic acid analog is used as a carrier. As illustrated in this drawing, the nucleic acid analog has a cationic group and a hydrophilic polymer as primary structures, and the nucleic acid to be delivered such as a natural nucleic acid has anionic properties. An assembly structure in which the anionic nucleic acid to be delivered and the cationic group of the nucleic acid analog are assembled with each other by electrostatic interaction is formed to form a complex (ion complex) composed of the nucleic acid analog as a carrier and the nucleic acid to be delivered.

Here, when there is complementarity between the nucleic acid to be delivered and the cationic group, a double strand is formed by hydrogen bonding between bases, but in the present disclosure, even when the complementarity is low, it is excellent in that the cationic group and the phosphate group of the nucleic acid to be delivered can assemble with each other through electrostatic interaction. That is, not only a nucleic acid to be delivered having complete complementarity (100% match) but also, for example, a nucleic acid to be delivered having complementarity of 80% or 90% forms an assembly structure by the electrostatic interaction to form a complex. Therefore, the nucleic acid to be delivered which is a non-complementary strand can be delivered to the target site. The degree of complementarity between the cationic artificial nucleic acid and the nucleic acid to be delivered is preferably 50% or more, more preferably 80% or more, and particularly preferably 100%, depending on the type of cationic group and the like.

In an aqueous environment, such as in blood, segments of the hydrophilic polymer of the complex assemble with each other to form a nanoscale nucleic acid delivery construct (hereinafter, it may be simply referred to as a "construct"). As the nanoscale construct, there is a construct such as a micelle or a vesicle in which a segment of an assembly structure is located on an inner side and a segment of a hydrophilic polymer is located on an outer side (see "Nano-construct formation" in the drawing). These micelles and vesicles form a spherical construct having a hollow portion in a central portion, and a medicine such as a low molecular weight compound can be enclosed in the hollow portion. This makes it possible to deliver not only the nucleic acid to be targeted but also a small molecule medicine. The diameter of the hollow portion is about 50 to 500 nm.

Note that the micelle has a structure in which the segment of the hydrophilic polymer is located in the outermost shell of the spherical construct, and the segment of the assembly structure is located at a position facing the hollow portion at the center of the spherical construct. On the other hand, the vesicle has a double membrane structure in which two complexes are assembled with each other via the segment of the assembly structure, and one segment of the hydrophilic polymer of the double membrane is located in the outermost shell of the spherical construct, and the other segment of the hydrophilic polymer is located at a position facing the hollow portion at the center of the spherical construct. For this reason, it is preferable to enclose a hydrophobic medicine or the like in the hollow portion of the micelle, and it is preferable to enclose a hydrophilic medicine or the like in the hollow portion of the vesicle.

Such a construct has high degradation stability because the nucleic acid to be delivered is located inside the spherical construct. In addition, since the construct has the segment of the hydrophilic polymer in the outermost shell, the construct is excellent in blood retention. Then, the construct is enclosed in an endosome in a cell or the like as a target site and taken up into cytoplasm, then escapes from the endosome, and releases a nucleic acid to be delivered or a low molecular medicine as a drug in the cytoplasm or nucleus (see "Biochemical evaluation" in the drawing).

Examples of use of such a construct include carriers for drug delivery against various diseases. For example, in Examples described below, it is illustrated that the nucleic acid analog of the present disclosure is effective as a carrier for delivering, as a nucleic acid to be delivered, microRNA-143 that is an anti-oncomicroRNA to K-Ras of a cancer promoting gene and a network thereof.

The surface of the nucleic acid delivery construct may be modified with various ligands. By modifying the surface of the construct with such a ligand, target directivity or the like can be imparted to the construct. For convenience, the nucleic acid delivery construct is sometimes referred to as "Reversiblly Ionic Oligonucleotide-based Nanoparticles," which may be abbreviated as "RION" or "RIO".

### (Method for producing nucleic acid analog)

Next, a method for producing a nucleic acid analog will be described. The nucleic acid analog can be produced by various methods, and examples thereof include a method of individually synthesizing a cationic artificial nucleic acid and a hydrophilic polymer and binding them. That is, the method for producing a nucleic acid analog includes:
(i) a cationic nucleic acid synthesis step of synthesizing a cationic artificial nucleic acid;
(ii) a hydrophilic polymer step of synthesizing a hydrophilic polymer; and
(iii) a binding step of binding the cationic artificial nucleic acid and the hydrophilic polymer.

More specifically, when a nucleic acid automatic synthesizer is used, the cationic artificial nucleic acid may first be synthesized by fixing the 3' or 5' of the nucleic acid, and then the hydrophilic polymer may be synthesized and conjugated with a ligand. Alternatively, the ligand may first be fixed, the hydrophilic polymer may then be synthesized, and the nucleic acid may be synthesized at the terminal end thereof, and a spacer may be inserted between the hydrophilic polymer and the nucleic acid. The nucleic acid thus produced is an anionic nucleic acid, but the nucleic acid produced by the above reaction can also be cationized.

This will be described in more detail.

### (a) Two-step synthesis method

The cationic artificial nucleic acid can be synthesized by a two-step reaction (two-step synthesis method). As the two-step synthesis method, mainly three kinds can be mentioned. Hereinafter, the synthesis methods will be sequentially described.

### (a-1) Two-step synthesis method I

In this method, sulfurization (S-conversion) of a nucleic acid is performed by automatic nucleic acid synthesis, and a hydrophilic polymer phosphoramidite (for example, ethylene glycol phosphoramidite) is linked to the sulfurized nucleic acid to synthesize an oligo PS-hydrophilic polymer, and then a Br compound is further reacted to introduce a cationic group into the nucleic acid (TEG-PS oligo and HEG-PS oligo systems of Examples described below).

In the case of a nucleotide backbone, the cationic artificial nucleic acid can be synthesized by a method including the steps of: introducing a thiophosphate ester into a linking structure; and reacting a bromo compound having a cationic group with the thiophosphate ester to introduce a cationic group into the linking structure, as described in Examples described below.

### (a-1-2) Introduction of thiophosphate ester

The thiophosphate ester can be synthesized by a known phosphoramidite method. As an outline of the phosphoramidite method, a nucleoside or a nucleotide in which 5' is protected with a 4,4'-dimethoxytrityl (DMTr) group is supported on a solid phase (supporting step). Next, the DMTr group is deprotected with a deprotection reagent such as dichloroacetic acid (deprotection step), and coupled with a phosphoramidite nucleotide in the presence of an activating agent such as 4,5-dicyanoimidazole (coupling step). Thereafter, the phosphite ester is converted into a thiophosphate ester by a sulfurizing agent such as (N,N-dimethylaminomethylidene)amino-3H-1,2,4-dithiazoline-3-thione (DDTT) (sulfurization step). Alternatively, the phosphite ester is converted into a phosphate diester by an oxidizing agent containing iodine, pyridine, or the like (oxidation step). By repeating this step, a synthetic nucleic acid containing a thiophosphate ester in the linking structure can be produced. By changing the type of the base of phosphoramidite, a synthetic nucleic acid having a desired sequence can be produced. Incidentally, it is also possible to introduce the thiophosphate ester only at a desired position of the linking structure constituting the nucleotide backbone by performing the sulfurization step instead of the oxidation step.

### (a-1-2)

Next, a bromo compound having an amine or an ammonium group is reacted with the obtained synthetic nucleic acid. Examples of the bromo compound include 3-bromo-1-propylamine hydrobromide, 2-bromo-N,N-diethylethylamine hydrobromide, (3-bromopropyl)trimethylammonium bromide, and the like The reaction between the synthetic nucleic acid and the bromo compound can be performed in a phosphate buffer or the like, and the reaction conditions can be appropriately set, but for example, the reaction can be performed at a pH within the range of 5 to 7, a reaction temperature of 30 to 60°C, and a reaction time of 10 to 50 hours.

### (a-2) Two-step synthesis method II

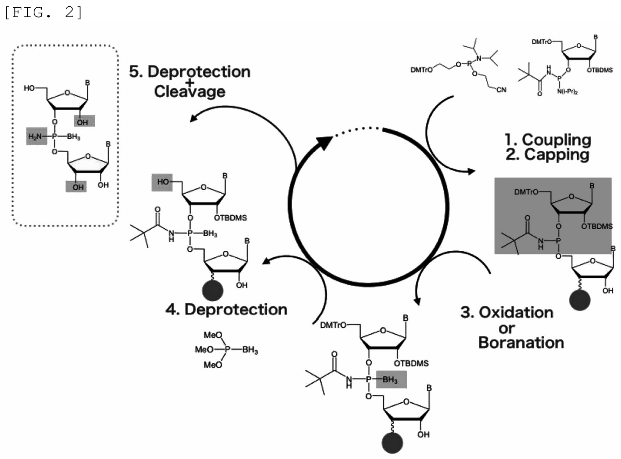

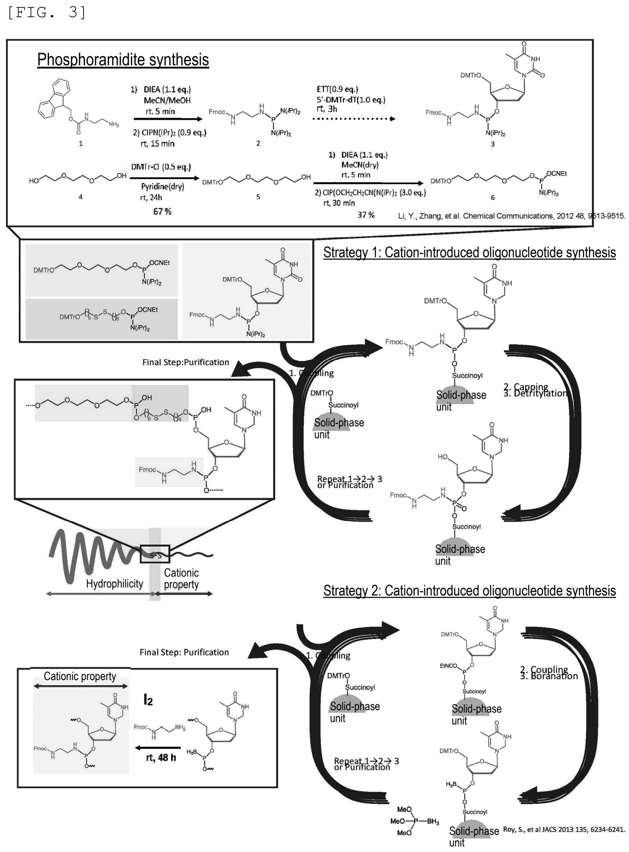

In this method, boranophosphation (B-conversion) of nucleic acid is performed by automatic nucleic acid synthesis, and a hydrophilic polymer phosphoramidite (for example, ethylene glycol phosphoramidite) is linked to the boranophosphated nucleic acid to synthesize an oligo B-hydrophilic polymer (Fig. 2). Thereafter, an amino group compound is further reacted by iodine oxidation to introduce a cationic group (strategy 2 in Fig. 3). In this method, it is also possible to introduce two cationic groups into one phosphate group (double cation introduction) by using a cationic phosphoramidite described below.

### (a-3) Two-step synthesis method III

In this method, a cationic artificial nucleic acid is synthesized by automatic nucleic acid synthesis, and then a hydrophilic portion is introduced by a click reaction (system of PEG-PMO in Examples described below). That is, it is a method in which a cationic artificial nucleic acid having a cationic group in the backbone is synthesized, and then a hydrophilic azide compound such as azide polyethylene glycol is linked thereto by a click reaction.

### (b) One-step synthesis method

The method described above is a two-step synthesis method in which a synthetic nucleic acid containing a thiophosphate ester is produced and a bromo compound is reacted therewith, but a cationic artificial nucleic acid can also be synthesized in one step. Fig. 2 illustrates a scheme of this method. The outline of this method is a one-step synthesis of a cation-hydrophilic polymer by automatic nucleic acid synthesis of cationic phosphoramidite and ethylene glycol phosphoramidite (strategy 1 in Figs. 2 and 3). This method is basically a scheme similar to the phosphoramidite method illustrated in the two-step synthesis method, but there are some differences.

First, a diisopropylamino phosphoramidite compound and a nucleotide monomer are reacted with the nucleotide supported on the solid phase ("1. Coupling" in the drawing). Some hydroxyl groups are protected with a protecting group ("2. Capping"), and oxidized or borated with an oxidizing agent or a borating agent ("3. Oxidation or Boranation"). An amino group compound is reacted by iodine oxidation to introduce a cationic group.

Fig. 3 illustrates an example of a synthesis scheme of a nucleic acid analog. In this example, a scheme for synthesizing a nucleic acid analog having a nucleotide backbone as a cationic artificial nucleic acid and polyethylene glycol as a hydrophilic polymer isillustrated. In "Phosphoramidite synthesis" in the drawing, a phosphoramidite nucleotide is synthesized by a known method in the order of compounds 1 to 3. On the other hand, phosphoramidite polyethylene glycol is synthesized in the order of compounds 4 to 6.

Next, a nucleic acid analog is synthesized by the scheme illustrated in "Strategy 1: Cation-introduced oligonucleotide synthesis" in the drawing. Specifically, a phosphoramidite nucleotide and a phosphoramidite polyethylene glycol synthesized by the above scheme, and phosphoramidite disulfide having a disulfide bond and phosphoramidite in the molecule are used as raw materials. Then, a reaction such as protection with a protecting group and deprotection is performed to bond the hydrophilic polymer to the 5'-position of the cationic artificial nucleic acid. The cationic artificial nucleic acid may be synthesized in one step by solid phase synthesis as in the scheme illustrated in "Strategy 2: Cation-introduced oligonucleotide synthesis" in the drawing. Reference can be made to Patent Literature 1 and the like for the method for producing a cationic artificial nucleic acid.

### (Method for producing nucleic acid delivery construct)

Next, a method for producing a nucleic acid delivery construct will be described. The nucleic acid delivery construct assembles a nucleic acid to be delivered with a nucleic acid analog to form a complex (assembly step). When there is complementarity between a cationic group of the nucleic acid analog and a phosphate group of the nucleic acid to be delivered, both are bonded by annealing to form a double strand. The ratio between the nucleic acid analog and the nucleic acid to be delivered at the time of annealing is not particularly limited, but is preferably within the range of nucleic acid analog: nucleic acid to be delivered = 1: 1 to 1: 10. Annealing is performed by raising the temperature to a predetermined temperature and then lowering the temperature. As the annealing, the temperature is preferably raised to 80°C or higher, and more preferably raised to 90°C or higher. Also, the retention time of the raised temperature is preferably 5 minutes or longer, and more preferably 10 minutes or longer. Thereafter, the temperature is lowered to 50°C or lower, preferably 30°C or lower, and the temperature is maintained for 10 minutes or longer, and preferably 30 minutes or longer.

From the viewpoint of electrostatic interaction between the cationic group of the nucleic acid analog and the phosphate group of the nucleic acid to be delivered, this step is preferably performed under a pH condition lower than the pKa of the cationic group of the nucleic acid analog and higher than the pKa of the phosphate group of the nucleic acid to be delivered. That is, under such pH conditions, the cationic group is positively charged and the phosphate group is negatively charged, and these groups are electrostatically bonded. Such pH conditions depend on the pKa of the cationic group and the like, but the pH is preferably about 2 to 7, and more preferably about 3 to 6.

Next, a plurality of complexes are assembled to form a higher order structure (assemble forming step). In this step, the complexes are self-aggregated in an aqueous solvent such as water or an aqueous solution to form a construct such as a micelle or a vesicle. The concentration of the complex for forming a construct is about 25 to 2,500 µM, and more preferably in the range of 100 to 1,000 µM.

### (Nucleic acid delivery method)

The nucleic acid delivery method of the present disclosure includes an administration step of administering the nucleic acid delivery construct to incorporate the nucleic acid delivery construct into a target site, and a releasing step of releasing the nucleic acid in the target site. In the administration step, the above-described construct in a state of being bound to the nucleic acid to be delivered is administered to a human, other mammals, or the like. A drug containing the construct is preferably administered into the blood, but the administration can be appropriately determined according to the disease to be treated, the type of hydrophilic polymer, and the like. The administered construct is taken up into cytoplasm via endosomes when the cell is the target site. In the releasing step, the nucleic acid to be delivered is released from the construct taken up into the cell as the target site. A construct in which the cationic group is highly pH-responsive such as quaternary ammonium is likely to collapse under an acidic environment in the endosome and release the nucleic acid to be delivered. As described above, it is also possible to enclose a low molecular medicine in the hollow portion of the construct and release the medicine in an evaluation site. By such a method, the nucleic acid to be delivered or the low molecular medicine can be delivered to the target site.

Hereinafter, the present disclosure will be specifically described with reference to Examples, but these Examples will not limit the object of the present disclosure. Further, in the following Examples, the expression "%" is on a mass basis (mass percent) unless otherwise particularly defined.

### Examples

### (Cells)

DLD-1 (a human colorectal cancer cell line), ASF4-1 (a human fibroblast cell line), PRMI8226 (a human multiple myeloma cell line), NB4 (a human acute promyelocytic leukemia pre-B cell line), HL-60 (a human promyelocytic leukemia cell line), and Jurkat (a human T-cell leukemia-derived cell line) used for the evaluation were obtained from the JCRB Cell Bank of the National Institutes of Biomedical Innovation, Health and Nutrition (NIBIOHN). For the culture of DLD-1, PRMI8226, NB4, HL-60, and Jurkat cells, RPMI-1640 medium (manufactured by FUJIFILM Wako Pure Chemical Corporation) was used. ASF4-1 cells were cultured in Eagle's minimum essential medium. All media were supplemented with 10 (v/v)% heat-inactivated fetal bovine serum (Nichirei Biosciences Inc.), and each cell line was incubated in an atmosphere of 95% air and 5% carbon dioxide.

### (Cell evaluation)

All cells were seeded onto 12-well plates at 0.25 × 10⁵ cells/well one day before sample treatment. Samples were administered at concentrations of miR-143#12 ranging from 0.5 to 10 nM. Cell evaluation was performed after culturing for 48 hours or 72 hours. Cell proliferation was evaluated by counting the number of viable cells using the trypan blue dye exclusion method. After culturing, cells were collected, and mRNA expression levels and protein expression levels were analyzed, respectively.

### (Particle size distribution)

The particle size distribution was determined by dynamic light scattering using a Zetasizer (Beckman Coulter Inc.). For the measurement, a 10 µM PBS solution (pH 7.4) of the nucleic acid to be delivered (miR-143 of the present disclosure) was prepared and used as a sample.

RNA was isolated from cultured cells using NucLeoSpin (registered trademark) miRNA (TaKaRa). The RNA concentration and purity were evaluated using UV spectrophotometry. RNU6B was used as an internal reference. In addition, qRT-PCT for evaluating the mRNA expression levels of NRAS and KRAS was performed using qPCT Thunderbird (registered trademark) Next XYBR (registered trademark) qPCR Mix (TOYOBO). The primers for NRAS, KRAS, and GAPDH are as follows. GADPH was used as an internal control. Relative expression levels were calculated using the ΔΔCt method.
NRAS sense strand: 5'-CCT CCT CAC TTG GCT GTC TG-3' (SEQ ID NO: 27)
NRAS antisense strand: 5'-TCA CGT TTG CGG TTT GGT TC-3' (SEQ ID NO: 28)
KRAS sense strand: 5'-TGG TGG TGT GCC AAG ACA TT-3' (SEQ ID NO: 29)
KRAS antisense strand: 5'-CAC CTC ACC ATG CCA TCT CA-3' (SEQ ID NO: 30)
GAPDH sense strand: 5'-TCT AGA CGG CAG GTC AGG TCC ACC-3' (SEQ ID NO: 31)
GAPDH antisense strand: 5'-CCACCC ATG GCA AAT TCC ATG GCA-3' (SEQ ID NO: 32)

### (Western blot)

The cell lysate was prepared using a lysis buffer composed of 10 mM Tris-HCl (pH 7.4), 1% NP-40, 0.1% deoxycholic acid, 0.1% SDS, 2% protease inhibitor cocktail, 2% phosphatase inhibitor cocktail II, and 2% phosphatase inhibitor cocktail III (Sigma-Aldrich), and was left on ice for 20 minutes. After centrifugation at 13,000 rpm (16,200 x g) at 4°C for 20 minutes, the supernatant was used as a protein sample. The protein concentration was measured using a DC Protein Assay Kit (Bio-Rad). 2 µg of solubilized protein was separated by sodium dodecyl sulfate polyacrylamide gel electrophoresis using a 10.0% or 12.5% polyacrylamide gel, and electroblotted onto an Immobilon-P polyvinylidene difluoride (PVDF) membrane (Merck Millipore). After blocking non-specific binding with 20% PVDF in distilled water using Can Get Signal (registered trademark: TOYOBO) for 1 hour, the Immobilon-P membrane was incubated overnight at 4°C with the primary antibody using Get Signal Solution 1 (TOYOBO). On the following day, the membrane was washed three times with Tris-buffered saline (TBS) containing 0.1% Tween 20 (TBS-T). The immunoblot was visualized using the Immobilon Forte Western horseradish peroxidase (HRP) substrate (Millipore). The immunoblot images were acquired using an ImageQuant LAS4000 biomolecular imager (GE Healthcare Life Sciences, Pittsburgh, PA, USA). Densitometric analysis was performed using the image analysis software ImageQuant Total Lab-7 (GE Healthcare Life Sciences).

The primary antibodies used were as follows:
Anti-AKT, ERK1/2, PARP, Cyclin D1, ERK5, SOS1, GLUT1, and GLUT4 (Cell Signaling Technology); anti-KRAS (LifeSpan BioScience, Seattle, WA, USA); and anti-total (T)-RAS, including KRAS, HRAS, and NRAS (abcam).

Anti-β-actin (Sigma-Aldrich); and anti-α-Tubulin (Medical & Biological Laboratories Co., Ltd., Tokyo, Japan).

**[Table 3]**

| **SEQ ID** | **Name** | **5' end** | **Oligonucleotide (5'→3')** | **3' end** |
|---|---|---|---|---|
| **1** | **G#12** | **-** | **U_{f}^Gₘ^A_{f}*Gₘ*A_{f}*Uₘ*G_{f}*Aₘ*A_{f}*Gₘ*C_{f}*Aₘ*C_{f}*Uₘ*G_{f}*Uₘ*A_{f}*Gₘ*C_{f}*Uₘ*C_{f}*Aₘ** | **^dT^dT** |
| **2** | **P#1** | **-** | **U*G*A*G*C*U*A*C*A*G*U*G*C*U*G*C*A*U*C*U*C*U** | |
| **3** | **P#2** | **-** | **U^G^A^G^C^U^A^C^A^G^U^G^C^U^G^C^A^U^C^U^C^U** | ***X*X*X*X*X*X*Y*Z** |
| **4** | **P#3** | **-** | **U*G*A*G*C*U*A*C*A*G*U*G*C*U*G*C*A*U*C*U*C*U** | ***X*X*X*X*X*Y*Glu** |
| **5** | **AS#1** | **-** | **G*G*C*C*U*U*U*C*A*C*U*A*C*U*C*C*U*A*C** | **^dT^dT** |
| **6** | **S#1** | **-** | **G^U^A^G^G^A^G^U^A^G^U^G^A^A^A^G^G^C^C** | **^t^t*X*X*X*X*X*X*Y*Z** |
| **7** | **S#2** | **-** | **G*U*A*G*G*A*G*U*A*G*U*G*A*A*A*G*G*C*C** | ***t*t*X*X*X*X*X*X*Y*Glu** |

G: guide strand of miR-143
P: artificial nucleic acid of the present disclosure
AS: antisense strand of siRNA control
S: sense strand of siRNA control
N: RNA
n: DNA
Mf: 2'-F-RNA
*: anionic backbone of phosphodiester
^: -P(S)OH-
+: cationic backbone of phosphate
X: hexaethylene glycol
Y: linker (spacer 2 of the present application)
Z: acetylene derivative
Glu: glucose
Underline: mismatched base

Note that the sequences and sequence numbers in the above tables in the sequence listing are as follows.

P#1 (SEQ ID NO: 21), P#2 (SEQ ID NO: 22), P#3 (SEQ ID NO: 23), AS#1 (SEQ ID NO: 24), S#1 (SEQ ID NO: 25), and S#2 (SEQ ID NO: 26)

### (Production Example 1: Synthesis of P#2)

The base sequences of the artificial nucleic acids (ID4 and P#3) for delivering miR-143 and its derivatives were designed based on the base sequences of the nucleic acids to be delivered. In the present example, miR-143 and its derivatives used were nucleic acids of G#12 (the same as AS-4).

The artificial nucleic acid P#2 was synthesized using an automatic nucleic acid synthesizer. After the nucleotide sequence described in P#2 was linked via thiophosphoric diester bonds, six units of hexaethylene glycol were introduced through phosphate groups by the phosphoramidite method, and a triple-bond construct represented by the following chemical formula was further constructed at the terminal end, thereby obtaining P#2 in which the artificial nucleic acid was linked via thiophosphoric diester bonds.

Separately, 1-azidoglucose was synthesized according to the method described in WO 2016/152980 A.

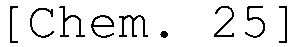

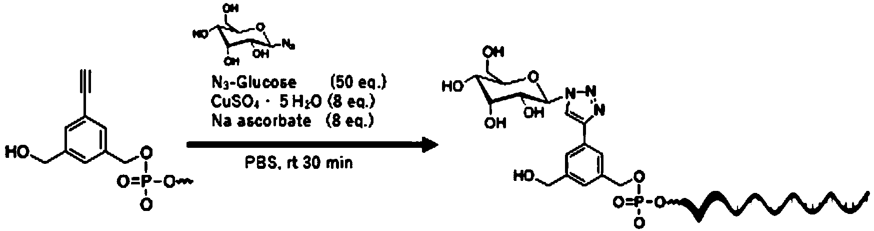

P#2 in which the artificial nucleic acid synthesized above was linked via thiophosphoric diester bonds was dissolved in PBS (pH 7.4). Next, a 50 mM sodium ascorbate solution (10 equivalents), a 50 mM copper(II) sulfate solution (10 equivalents), and a 50 mM 1-azidoglucose solution were mixed with nucleic acid P#2 (10 nmol, 1 equivalent) in PBS (pH 7.4), and the mixture was allowed to react for 15 minutes. After the reaction, the sample was purified by high-performance liquid chromatography, thereby obtaining nucleic acid P#3 in which the artificial nucleic acid was linked via thiophosphoric diester bonds.

The nucleic acid P#3 (4 nmol) in which the artificial nucleic acid was linked via thiophosphoric diester bonds and 4 M 2-(diethylamino)ethyl bromide hydrobromide (Waco, Osaka, Japan, 2 µL, 8 mmol) were reacted in PBS at 45°C for 24 hours. After the reaction, the sample was dialyzed with distilled water for 3 to 5 days, and purified by lyophilization, thereby obtaining P#3.

### (Production Example 2)

P#3 was obtained in the same manner as in Production Example 1, except that it was an artificial nucleic acid having a terminal triple bond represented by the following chemical formula and linked via thiophosphoric diester bonds in Production Example 1.

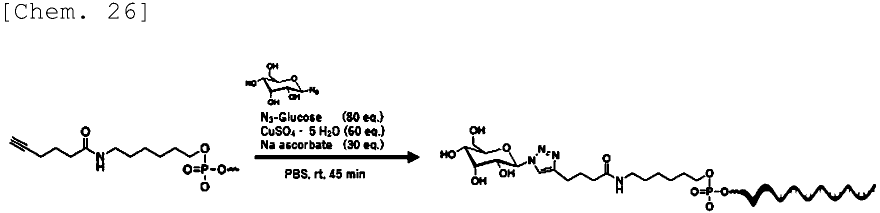

### (Production Example 3: Glu-RION)

miR-143 analog G#12 (which may also be referred to as "miR-143G#12") and the nucleic acid analog P#3 obtained in Production Example 1 were annealed at a ratio of 1:5 at 98°C for 15 minutes, 25°C for 50 minutes, and 45°C for 50 minutes to obtain Glu-RION-miR143#12.

### (Measurement of particle size distribution)

miR-143 analog G#12 and the nucleic acid analog P#3 obtained in Production Example 1 were mixed at a molar ratio of 1:5 and annealed. The results are illustrated in Fig. 1c. It was confirmed that miR-143G#12 and the nucleic acid analog P#3 were annealed, self-assembled to form RION-miR143#12 nanoparticles.

### (Reference Example 1)

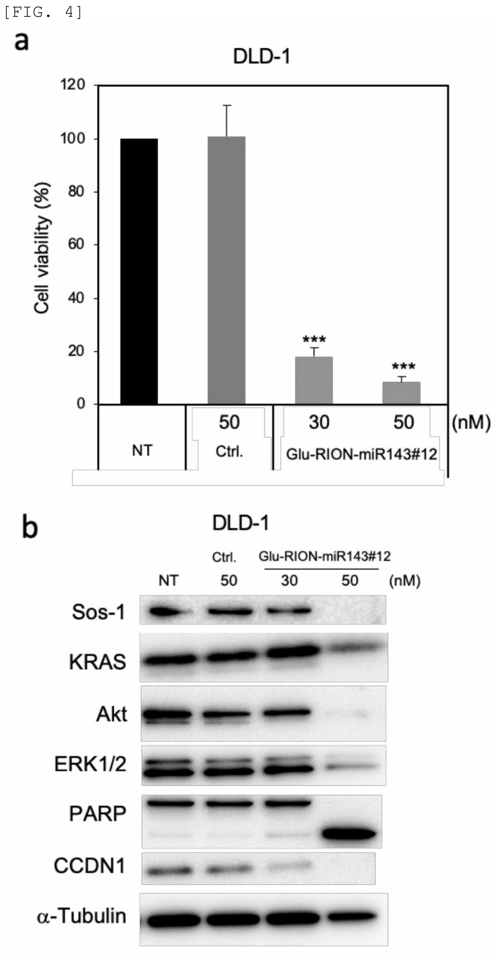

The effect of Glu-RION-miR143#12 on human colorectal cancer DLD-1 cells was examined and compared with that of RION-Control (Cntl.). The results are illustrated in Fig. 4 and Table 4. The cellular uptake of Glu-RION-miR143#12 was improved compared with that of the construct without glucose modification (Figs. 4a and 4b). While RION-miR143#12 enters cells via passive transport, Glu-RION-miR#143 is considered to exhibit enhanced cellular uptake through glucose transporters expressed in the cells. The IC₅₀ of Glu-RION-miR143#12 was 3.3 nM, and the expression of KRAS, Sos-1, Akt, and ERK1/2, which are target genes of miR-143, was suppressed. The cell death of DLD-1 was demonstrated to be apoptosis, as cleaved PARP-1 was observed. Accordingly, it is considered that nucleic acid introduction into cancer cells through glucose transporters is useful.

### (Example 1)

The characteristics of human KRAS-mutant hematologic malignancy cells and the growth-inhibitory effect by delivery of miR-143#12 using Glu-RION-miR143#12 were examined.

As hematologic malignancy cells, RPMI8226 (KRASG12A), NB4 (KRASA18D), HL-60 (NRASQ61L), and Jurkat (wild type) were used. These are suspension cells in which nucleic acid introduction is considered to be difficult by conventional transfection methods. The results are illustrated in Fig. 5 and Table 4. The IC50 was 6.9 nM for PRMI8226, the IC50 was 6.4 nM for NB4, and IC50 was 7.3 nM for HL-60.

In PRMI8226 cells, the effect of Glu-RION-miR143#12 was confirmed, whereas no cell growth-inhibitory effect was observed when Glu-RION without miR-143#12 was used or when miR-143#12 was delivered using the gene transfection reagent Lipofectamine (Fig. 5b). In particular, although lipofection reagents are widely used as general gene transfection reagents, it is known that the transfection efficiency is extremely low in suspension cells such as PRMI8226 cells. In Fig. 5b, even at a nucleic acid concentration of 200 nM, cell death was not induced by the introduction of miR143#12. On the other hand, Glu-RION-miR143#12 in Fig. 5c induced cell death in a concentration-dependent manner at concentrations of 1 to 10 nM. Accordingly, Glu-RION-miR143#12 into which nucleic acids are introduced PRMI8226 cells is a useful nucleic acid introduction technology because nucleic acid introduction can be achieved at low concentrations. The cell growth-inhibitory effect was also confirmed in HL-60 cells and NB4 cells, and apoptosis was observed in NB4 cells (Fig. 5d). It was confirmed that glucose transporters (GLUT1 and GLUT4) were expressed in hematologic malignancy cells (Fig. 5g).

### (Example 2)

The effect of miR-143#12 delivery into hematologic malignancies using Glu-RION-miR143#12 was examined.

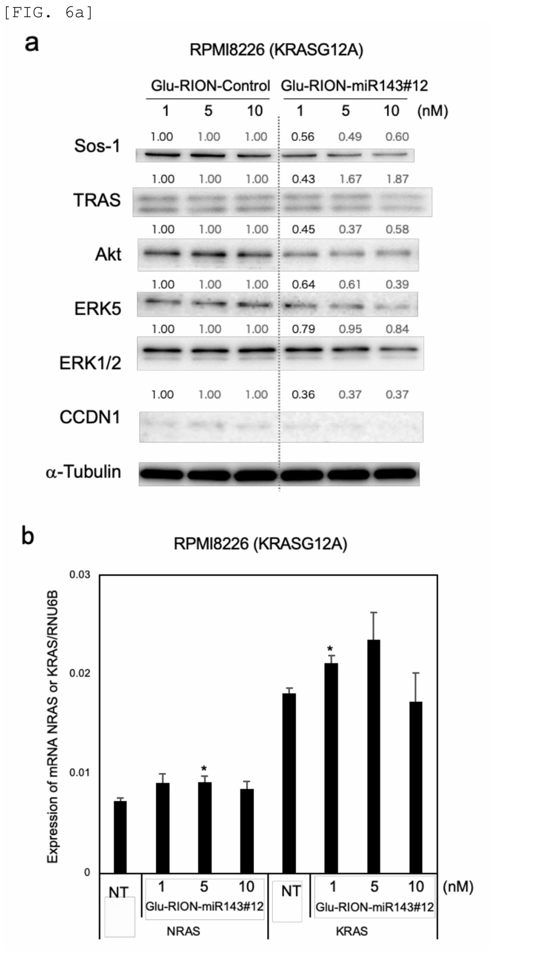

In RPMI8226 cells, the expression levels of RAS and RAS-related proteins (Sos-1, ERK1/2, ERK5, and Akt) were determined by Western blotting. The results are illustrated in Fig. 6a. These results indicate that the expression of the target genes of miR-143, TRAS, Akt, ERK5, and ERK1/2, was suppressed by miR-143#12 through the use of Glu-RION-miR143#12. This is considered to be due to the delivery of miR-143#12 into the cells, resulting in suppression of protein expression by RNA interference. In RPMI8226 cells, a glucose transporter, which is one of the nutrient transmitters, is present, and it is considered that nucleic acids were efficiently taken up into the suspended cells because the ligand present on the surface of the RION recognized this transporter.

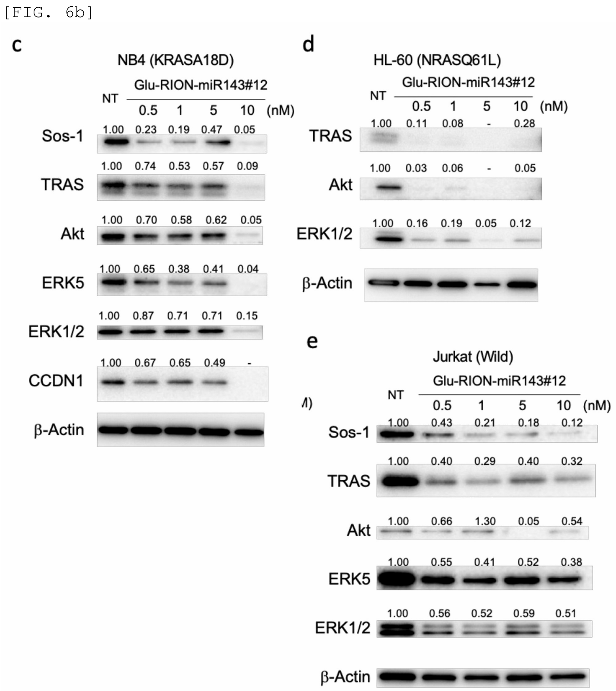

In Fig. 6b, the expression level of mRNA was evaluated. It is known that miRNA inhibits protein translation by binding to the untranslated region of mRNA, that is, the expression level of mRNA itself does not decrease by the action of miRNA. When the expression levels of mRNA NRAS or KRAS were analyzed, no significant decrease in expression was observed. These findings also suggest that the inhibitory effects of Glu-RION-miR143#12 on protein expression and cell proliferation in RPMI8226 cells were attributable to the action of miRNA143#12. In Figs. 6c and 6d, the protein expression levels of Glu-RION143#12 in NB4 and HL60 cells were evaluated. As a result, in NB4, decreased expression of the target proteins of miR143#12, Sos-1, T-RAS, Akt, ERK5, and ERK1/2, was observed; in HL-60, decreased expression of T-RAS, Akt, and ERK1/2 was observed, suggesting that the inhibition of cell proliferation (Fig. 5e) was induced by the pharmacological action of miR143#12. On the other hand, in Fig. 6e, the protein expression level in Jurkat was evaluated. In Jurkat, decreased expression of the target proteins of miR143#12, Sos-1, T-RAS, Akt, ERK5, and ERK1/2, was observed. However, since inhibition of cell proliferation was not induced in Jurkat (Fig. 6e), it was inferred that Jurkat is not a cancer in which RAS functions as a driver gene.

### (Example 3)

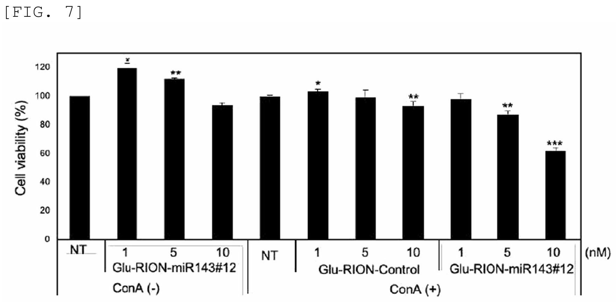

Using Glu-RION-miR143#12, the difference in stimulation of peripheral lymphocytes under the steady-state condition of miR-143#12 was examined between cases with and without concanavalin A. The results are illustrated in Fig. 7. The survival rate of peripheral lymphocytes increased when concanavalin A was not used, but when concanavalin A was used, the proliferation of peripheral lymphocytes tended to be inhibited. This growth inhibition is considered to be due to cell cycle arrest.

**[Table 4]**

| **Cell line** | **RAS mutation** | **Cell type** | **Effect profiles by miR143#12 delivery** | | |
|---|---|---|---|---|---|
| | | | **IC₅₀ (nM)** | **Cell status** | **Protein downregulation status** |
| **RPMI8226** | **KRASG12A** | **B-cell** | **6.9** | **Cell cycle arrest and apoptosis** | **Sos-1, KRAS, NRAS, Akt, ERK5, and ERK1/2** |
| **NB4** | **KRASA18D** | **Myeloid** | **6.4** | **Cell cycle arrest and apoptosis** | **Sos-1, KRAS, NRAS, Akt, ERK5, and ERK112** |
| **HL-60** | **NRASQ61L** | **Myeloid** | **7.3** | **Cell cycle arrest** | **KRAS, NRAS, Akt, and ERK1/2** |
| **Jurkat** | **NRAS, KRAS Wild** | **T-cell** | **-** | **-** | **Sos-1, KRAS, NRAS, Akt, ERKS, and ERK1/2** |
| **DLD-1** | **KRASG13D** | **Colon cancer** | **3.3** | **Cell cycle arrest and apoptosis** | **Sos-1, KRAS, NRAS, Akt, ERK5, and ERK112** |
| **ASF4-1** | **Wild** | **Skin fibroblasts** | **-** | **-** | **-** |

## Claims

1. Use of a nucleic acid delivery construct for treatment of a hematologic malignancy, the nucleic acid delivery construct having an assembly structure in which a nucleic acid analog represented by the following formula (1) and a nucleic acid to be delivered containing a microRNA that regulates a network of the oncogene KRAS are assembled with each other through electrostatic interaction:
[Chem. 1] **L-(S2)s-H-**(S1)t-N **(1**)
wherein N represents a cationic artificial nucleic acid, H represents a hydrophilic polymer, S1 represents a spacer 1, S2 represents a spacer 2, L represents a ligand, s represents 0 or 1, and t represents 0 or 1;
N has structural units in which a base is bonded to a hexose selected from ribose and deoxyribose, linking structures linking two of the structural units, and a backbone structure having cationic groups; and
the cationic artificial nucleic acid is capable of assembling through electrostatic interaction between a phosphate group of the nucleic acid to be delivered and the cationic group.

2. The use of a nucleic acid delivery construct for treatment of a hematologic malignancy according to claim 1, wherein a pKa of the cationic group is within a range of 6 to 9.

3. The use of a nucleic acid delivery construct for treatment of a hematologic malignancy according to claim 1, wherein the cationic group, in a cationic state, has a partial structure selected from the group consisting of the following formulas (C1) to (C7): 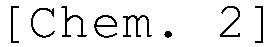 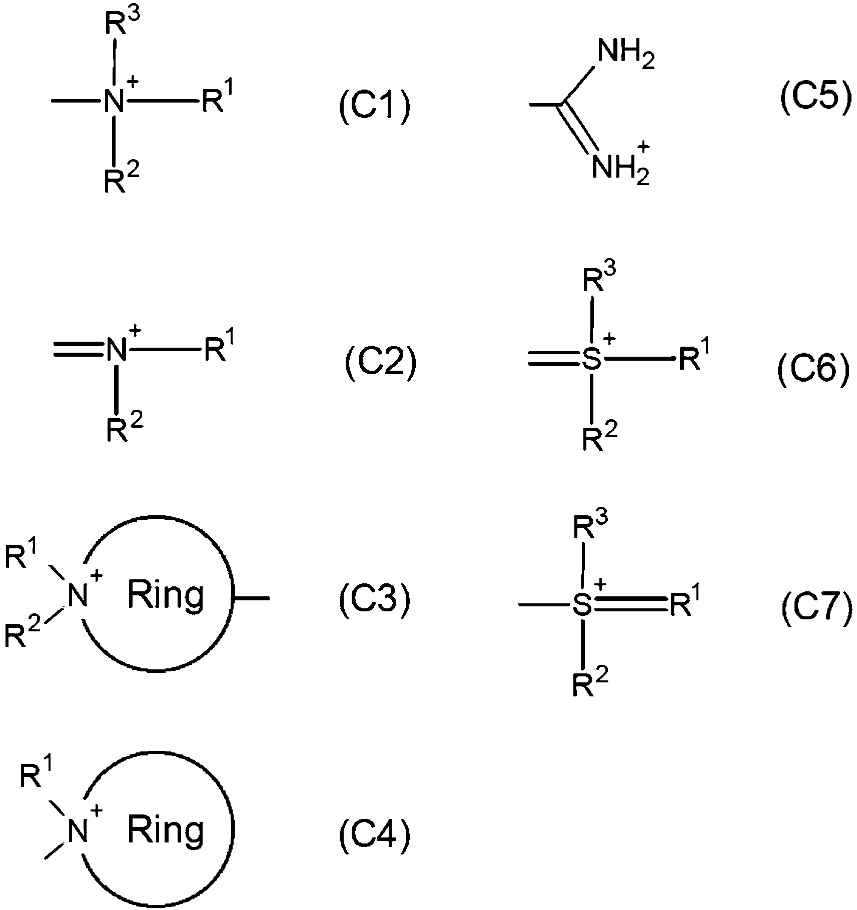 wherein R¹ to R³ represent hydrogen or an alkyl group having 1 to 10 carbon atoms, R₁ to R₃ may be the same as or different from each other, and Ring is a cyclic compound including 4 to 8 carbon atoms, and may be a heterocyclic ring in which one or more of the carbon atoms are substituted with a heteroatom selected from nitrogen, oxygen, and sulfur.

4. The use of a nucleic acid delivery construct for treatment of a hematologic malignancy according to claim 1, wherein the linking structure of the cationic artificial nucleic acid, in a cationic state, has at least a structure selected from the following formulas (L1) to (L4): 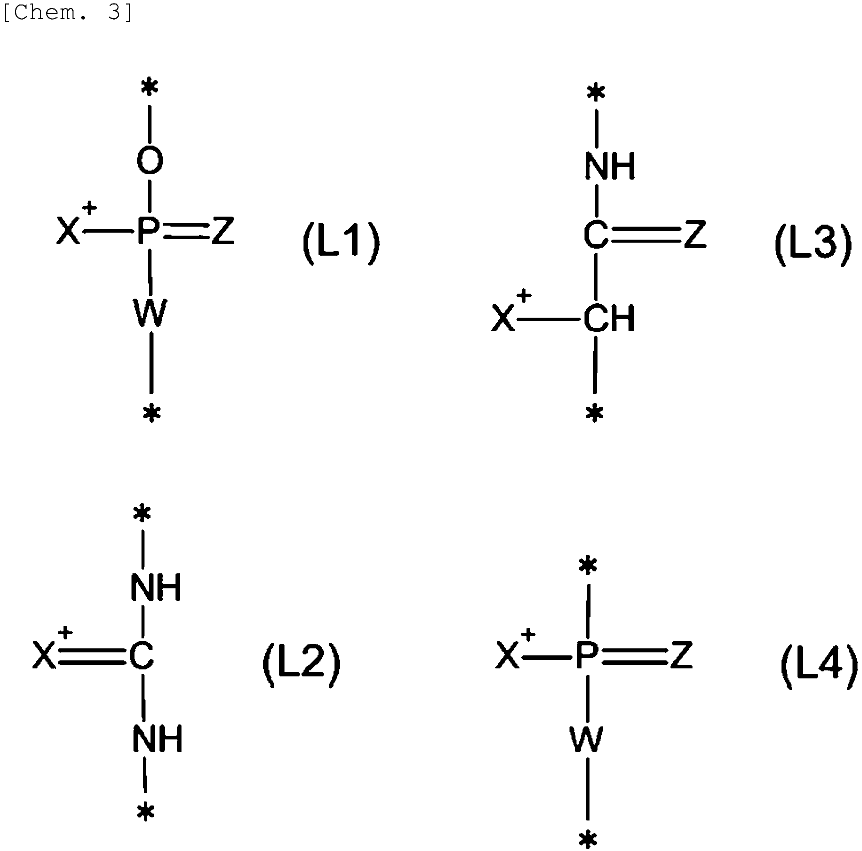 wherein X⁺ is a functional group containing the cationic group, Z represents O or S, W represents -O- or -NR₄-, wherein R₄ represents hydrogen or an alkyl group having 1 to 10 carbon atoms, and * denotes a bond with the structural unit adjacent thereto.

5. The use of a nucleic acid delivery construct for treatment of a hematologic malignancy according to claim 1, wherein the cationic artificial nucleic acid has a nucleotide backbone represented by the following formula (N1): 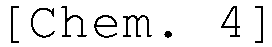 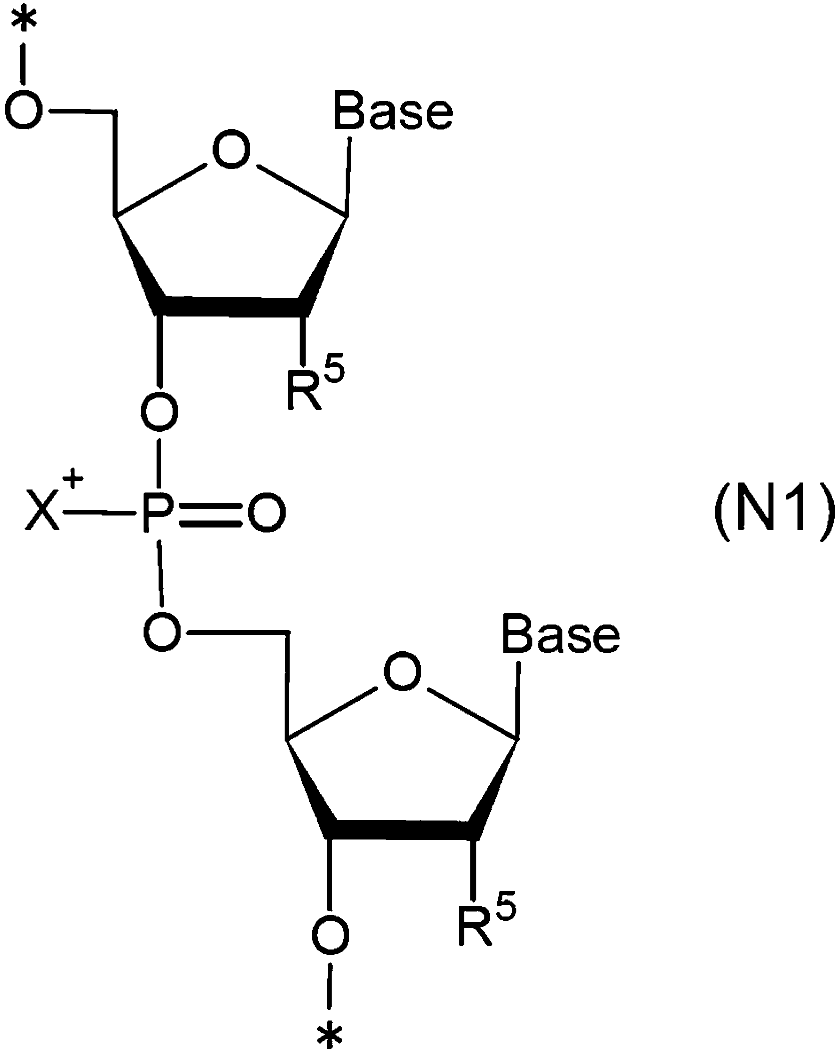 wherein X⁺ is a functional group containing the cationic group, Base represents a base, R₅ represents H or OH, * denotes a bond with a phosphate of an adjacent nucleotide backbone, at least one of * is bonded to the hydrophilic polymer at the 5' end or 3' end, and when not bonded to the hydrophilic polymer, * is hydrogen.

6. The use of a nucleic acid delivery construct for treatment of a hematologic malignancy according to claim 5, wherein X⁺, in a cationic state, is capable of being an ammonium cation represented by the following formula (F1): 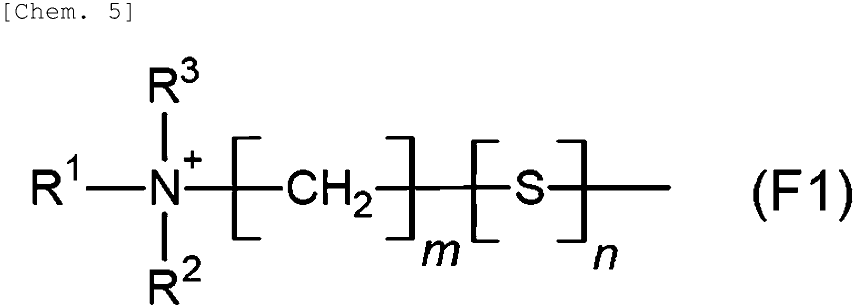 wherein R₁ to R₃ represent hydrogen or an alkyl group having 1 to 10 carbon atoms, and may be the same as or different from each other, m represents an integer between 0 and 10, and n represents an integer of 0 or 1.

7. The use of a nucleic acid delivery construct for treatment of a hematologic malignancy according to claim 1, wherein the hydrophilic polymer is selected from polyethylene glycol, polyvinyl alcohol, polyglutamic acid, polyvinylpyrrolidone, polyacrylamide, polyethyleneimine, polyalkyl acrylate, polyoxazoline, polyacrylamide, poly(carboxybetaine methacrylate), poly(sulfobetaine methacrylate), poly(2-methacryloyloxyethyl phosphocholine), hyaluronic acid, chitosan, dextran, and derivatives thereof.

8. The use of a nucleic acid delivery construct for treatment of a hematologic malignancy according to claim 7, wherein the hydrophilic polymer has a polyethylene glycol backbone represented by the following formula (A1): 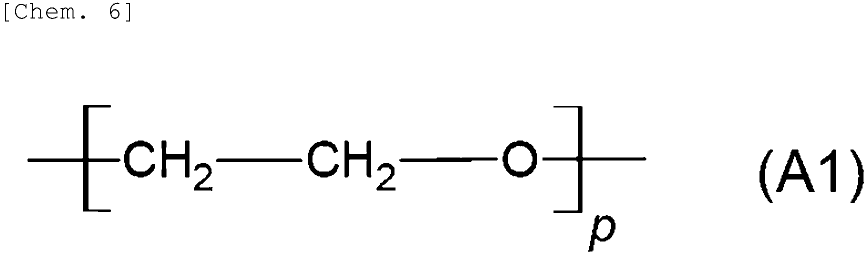 wherein p represents an integer between 1 and 20.

9. The use of a nucleic acid delivery construct for treatment of a hematologic malignancy according to claim 8, wherein the formula (A1) is linked through a phosphodiester group.

10. The use of a nucleic acid delivery construct for treatment of a hematologic malignancy according to claim 1, wherein the spacer 1 has a bond represented by the following formula (S11):
[Chem. 7] **-O-R⁶-S-S-R⁷-O- (S11)**
wherein R₆ and R₇ in the formula represent a methylene group having 1 to 12 carbon atoms, and R₆ and R₇ may be the same as or different from each other.

11. The use of a nucleic acid delivery construct for treatment of a hematologic malignancy according to claim 1, wherein the spacer 2 is a phosphodiester bond or a phosphodiester bond including a triazole represented by the following formula (S21): 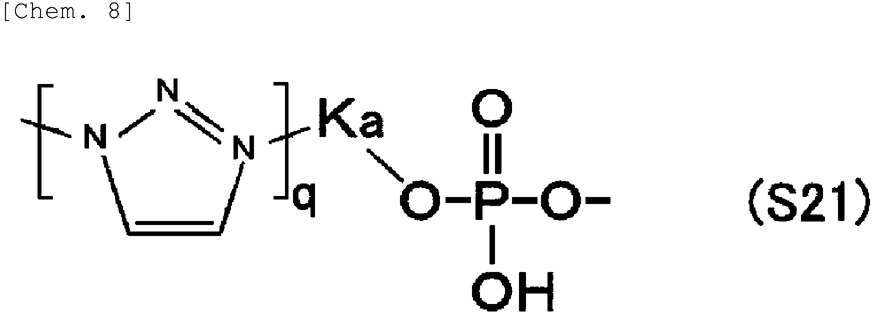 in the formula, Ka represents an amide bond containing a methylene group having 1 to 20 carbon atoms, a compound containing an aromatic ring having 6 to 12 carbon atoms, or a direct bond, q is 0 or 1, and when q is 0, the 1,2,3-triazole ring is bound to the ligand.

12. The use of a nucleic acid delivery construct for treatment of a hematologic malignancy according to claim 1, wherein the ligand is selected from glucose, mannose, galactose, sucrose, maltose, and lactose.

13. The use of a nucleic acid delivery construct for treatment of a hematologic malignancy according to claim 1, wherein the nucleic acid delivery construct is a nanoscale construct in which a plurality of nucleic acid delivery constructs are assembled with each other.

14. The use of a nucleic acid delivery construct for treatment of a hematologic malignancy according to claim 13, wherein the nucleic acid delivery construct is a vesicle or a micelle in which the assembly structure is located on an inner side and the hydrophilic polymer and the ligand are located on an outer side.

15. The use of a nucleic acid delivery construct for treatment of a hematologic malignancy according to claim 1, wherein the nucleic acid to be delivered is selected from miR-143 or an analog thereof.

16. The use of a nucleic acid delivery construct for treatment of a hematologic malignancy according to claim 15, wherein the miR-143 is selected from the group consisting of the following SEQ-1 to SEQ-23:
**[Table 1]**
| | Strand name | | 5'-end modification | Oligonucleotide (5' → 3') | 3'-end modification |
|---|---|---|---|---|---|
| **SEQ-1** | Sense strand | **S-1** | | U*G*A*G*G*U*G*C*A*G*U*G*C*U*G*C*A* U*C*U*C*U*G*G | |
| | Antisense strand | **AS-4** | | Uf^Gm^Af*Gm*Af*Um*Gf*Am*Af*Gm*Cf*Am *Cf*Um*Gf*Um*Af*Gm*Cf*Um*Cf*Am | **^dT^dT** |
| **SEQ-2** | Sense strand | **S-1** | | U*G*A*G*G*U*G*C*A*G*U*G*C*U*G*C*A* U*C*U*C*U*G*G | |
| | Antisense strand | **AS-7** | **-P(O)(OH)** | Uf^Gm^Af*Gm*Af*Um*Gf*Am*Af*Gm*Cf*Am *Cf*Um*Gf*Um*Af*Gm*Cf*Um*Cf*Am | **^dT^dT** |
| **SEQ-3** | Sense strand | **S-17** | | Uf^G^A*G*G*Uf*G*Cf*A*G*Uf*G*Cf*Uf*G*Cf *A*Uf*Cf*Uf*Cf*Uf*G*G | |
| | Antisense strand | **AS-4** | | Uf^Gm^Af*Gm*Af*Um*Gf*Am*Af*Gm*Cf*Am *Cf*Um*Gf*Um*Af*Gm*Cf*Um*Cf*Am | |
| **SEQ-4** | Sense strand | **S-1** | | U*G*A*G*G*U*G*C*A*G*U*G*C*U*G*C*A* U*C*U*C*U*G*G | |
| | Antisense strand | **AS-30** | | Um^Gm^Am*G*A*U*Gm*Am*Am*G*C*A*Cm*Um* Gm*U*A*G*Cm*Um*Cm*Am | **^dT^dT** |
| **SEQ-5** | Sense strand | **S-1** | | U*G*A*G*G*U*G*C*A*G*U*G*C*U*G*C*A* U*C*U*C*U*G*G | |
| | Antisense strand | **AS-31** | | Uf^Gf^Af*Gm*Am*Um*Gf*Af*Af*Gm*Cm* Am*Cf*Uf*Gf*Um*Am*Gm*Cf*Uf*Cf*Af | **^dT^dT** |
| **SEQ-6** | Sense strand | **S-17** | | Uf^G^A*G*G*Uf*G*Cf*A*G*Uf*G*Cf*Uf*G*Cf *A*Uf*Cf*Uf*Cf*Uf*G*G | |
| | Antisense strand | **AS-3** | | Uf^G^A*G*A*Uf*G*A*A*G*Cf*A*Cf*Uf* G*Uf*A*G*Cf*Uf*Cf*A | **^dT^dT** |
| **SEQ-7** | Sense strand | **S-17** | | Uf^G^A*G*G*Uf*G*Cf*A*G*Uf*G*Cf*Uf*G*Cf *A*Uf*Cf*Uf*Cf*Uf*G*G | |
| | Antisense strand | **AS-10** | | Uf^G■^Af*G■*Af*U■*Gf*A■*Af*G■*Cf*A■ *Cf*U■*Gf*U■*Af*G■*Cf*U■*Cf*A■ | **^dG^dG** |
| **SEQ-8** | Sense strand | **S-18** | | Um^G^A*G*G*Um*G*Cm*A*G*Um*G*Cm*Um* G*Cm*A*Um*Cm*Um*Cm*Um*G*G | |
| | Antisense strand | **AS-12** | | Uf^Gm^Af*Gm*Af*Um*Gf*Am*Af*Gm*Cf*Am*Cf*Um *Gf*Um*Af*Gm*Cf*Um*Cf*Am | **^Gₘ^Gₘ** |
| **SEQ-9** | Sense strand | **S-18** | | Um^G^A*G*G*Um*G*Cm*A*G*Um*G*Cm*Um* G*Cm*A*Um*Cm*Um*Cm*Um*G*G | |
| | Antisense strand | **AS-13** | | Uf^Gm^Af^Gm*Af*Um*Gf*Am*Af*Gm*Cf*Am*Cf*Um *Gf*Um*Af*Gm*Cf*Um*Cf*Am | **^G_{f}^G_{f}** |
| **SEQ-10** | Sense strand | **S-19** | | Uf^G^A*G*G*Uf*G*C*A*G*Uf*G*C*Uf*G*C* A*Uf*C*Uf*C*Uf*G*G | |
| | Antisense strand | **AS-3** | | Uf^G^A*G*A*Uf*G*A*A*G*Cf*A*Cf*Uf* G*Uf*A*G*Cf*Uf*Cf*A | **^dT^dT** |
| **SEQ-11** | Sense strand | **S-1** | | U*G*A*G*G*U*G*C*A*G*U*G*C*U*G*C*A* U*C*U*C*U*G*G | |
| | Antisense strand | **AS-42** | **VP-** | Um^Gm^Af*Gm*Af*Um*Gf*Am*Af*Gm*Cf Am*Cf*Um*Gf*Um*Af*Gm*Cf*Um*Cf*Am | **^dT^dT** |
| **SEQ-12** | Sense strand | **S-1** | | U*G*A*G*G*U*G*C*A*G*U*G*C*U*G*C*A* U*C*U*C*U*G*G | |
| | Antisense strand | **AS-47** | | Uf^Gm^Af*Gm*Af*Um*Gf*Am*Af*Gm*Cf*Am* Cf*Um*Gf*Um*Af*Gm*Cf*Um*Ab*Ab | **^dT^dT** |
**[Table 2]**
| | Strand name | | 5'-end modification | Oligonucleotide (5' → 3') | 3'-end modification |
|---|---|---|---|---|---|
| **SEQ-13** | Sense strand | **S-18** | | Um^G^A*G*G*Um*G*Cm*A*G*Um*G*Cm*Um* G*Cm*A*Um*Cm*Um*Cm*Um*G*G | |
| | Antisense strand | **AS-47** | | Uf^Gm^Af*Gm*Af*Um*Gf*Am*Af*Gm*Cf*Am* Cf*Um*Gf*Um*Af*Gm*Cf*Um*Ab*Ab | **^dT^dT** |
| **SEQ-14** | Sense strand | **S-1** | | U*G*A*G*G*U*G*C*A*G*U*G*C*U*G*C*A* U*C*U*C*U*G*G | |
| | Antisense strand | **A5-50** | **-P(O)(OH)₃** | Uf^Gm^Af*Gm*Af*Um*Gf*Am*Af*Gm*Cf*Am* Cf*Um*Gf*Um*Af*Gm*Cf*Um*Cf*Am | **^dG^dG** |
| **SEQ-15** | Sense strand | **S-1** | | U*G*A*G*G*U*G*C*A*G*U*G*C*U*G*C*A* U*C*U*C*U*G*G | |
| | Antisense strand | **AS-51** | **-P(O)(OH)₂** | Uf^Gm^Af*Gm*Af*Um*Gf*Am*Af*Gm*Cf*Am* Cf*Um*Gf*Um*Af*Gm*Cf*Um*Cf*Am | **^Gₘ^Gₘ** |
| **SEQ-16** | Sense strand | **S-1** | | U*G*A*G*G*U*G*C*A*G*U*G*C*U*G*C*A* U*C*U*C*U*G*G | |
| | Antisense strand | **AS-52** | **-P(O)(OH)₂** | Uf^Gm^Af*Gm*Af*Um*Gf*Am*Af*Gm*Cf*Am* Cf*Um*Gf*Um*Af*Gm*Cf*Um*Cf*Am | **^G_{f}^G_{f}** |
| **SEQ-17** | Sense strand | **S-1** | | U*G*A*G*G*U*G*C*A*G*U*G*C*U*G*C*A* U*C*U*C*U*G*G | |
| | Antisense strand | **AS-55** | **-P(O)(OH)₂** | Uf^Gf^Af*Gm*Am*Um*Gf*Af*Af*Gm*Cm*Am* Cf*Uf*Gf*Um*Am*Gm*Cf*Uf*Cf*Af | **^dT^dT** |
| **SEQ-18** | Sense strand | **S-1** | | U*G*A*G*G*U*G*C*A*G*U*G*C*U*G*C*A* U*C*U*C*U*G*G | |
| | Antisense strand | **AS-56** | **-P(O)(OH)₂** | Uf^Gm^Af*Gm*Af*Um*Gf*Am*Af*Gm*Cf*Am* Cf*Um*Gf*Um*Af*Gm*Cf*Um*Ab*Ab | **^dT^dT** |
| **SEQ-19** | Sense strand | **S-17** | | Uf^G^A*G*G*Uf*G*Cf*A*G*Uf*G*Cf*Uf*G*Cf *A*Uf*Cf*Uf*Cf*Uf*G*G | |
| | Antisense strand | **AS-56** | **-P(O)(OH)₂** | Uf^Gm^Af*Gm*Af*Um*Gf*Am*Af*Gm*Cf*Am* Cf*Um*Gf*Um*Af*Gm*Cf*Um*Ab*Ab | **^dT^dT** |
| **SEQ-20** | Sense strand | **S-18** | | Um^G^A*G*G*Um*G*Cm*A*G*Um*G*Cm*Um* G*Cm*A*Um*Cm*Um*Cm*Um*G*G | |
| | Antisense strand | **AS-56** | **-P(O)(OH)₃** | Ur^Gm^Af*Gm*Af*Um*Gf*Am*Af*Gm*Cf*Am* Cf*Um*Gf*Um*Af*Gm*Cf*Um*Ab*Ab | **^dT^dT** |
| **SEQ-21** | Sense strand | **S-1** | | U*G*A*G*G*U*G*C*A*G*U*G*C*U*G*C*A* U*C*U*C*U*G*G | |
| | Antisense strand | **AS-57** | **-P(O)(OH)₃** | Um^Gf^Am*Gf^Am*Uf^Gm*Af^Am*Gf^Cm*Af^ Cm*Uf^Gm*Uf^Am^Gf^Cm^Uf^Cm^Af | **^Aₘ^Aₘ** |
| **SEQ-22** | Sense strand | **S-17** | | Uf^G^A*G*G*Uf*G*Cf*A*G*Uf*G*Cf*Uf*G*Cf *A*Uf*Cf*Uf*Cf*Uf*G*G | |
| | Antisense strand | **AS-57** | **-P(O)(OH)₃** | Um^Gf^Am*Gf^Am*Uf^Gm*Af^Am*Gf^Cm*Af^ Cm*Uf^Gm*Uf^Am^Gf^Cm^Uf^Cm^Af | **^Aₘ^Aₘ** |
| **SEQ-23** | Sense strand | **S-18** | | Um^G^A*G*G*Um*G*Cm*A*G*Um*G*Cm*Um* G*Cm*A*Um*Cm*Um*Cm*Um*G*G | |
| | Antisense strand | **AS-57** | **-P(O)(OH)₂** | Um^Gf^Am*Gf^Am*Uf^Gm*Af^Am*Gf^Cm*Af^ Cm*Uf^Gm*Uf^Am^Gf^Cm^Uf^Cm^Af | **^Aₘ^Aₘ** |
in the sequences, A, U, G, and C each represent RNA containing adenine, uracil, guanine, or cytosine, dT and dG each represent DNA containing thymine or guanine, RNAf represents 2'-FRNA, RNAm represents 2'-OMeRNA, ^ represents -P(S)OH-, * represents -P(O)OH-, and Ab(Abasic) represents a group illustrated below: 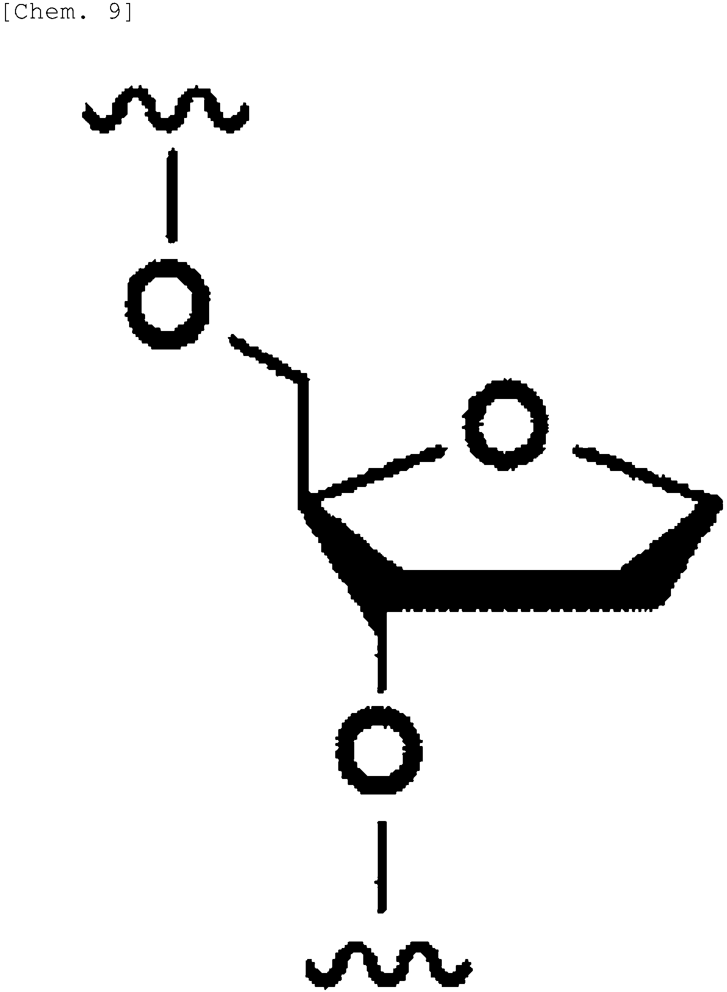 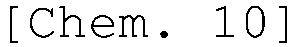 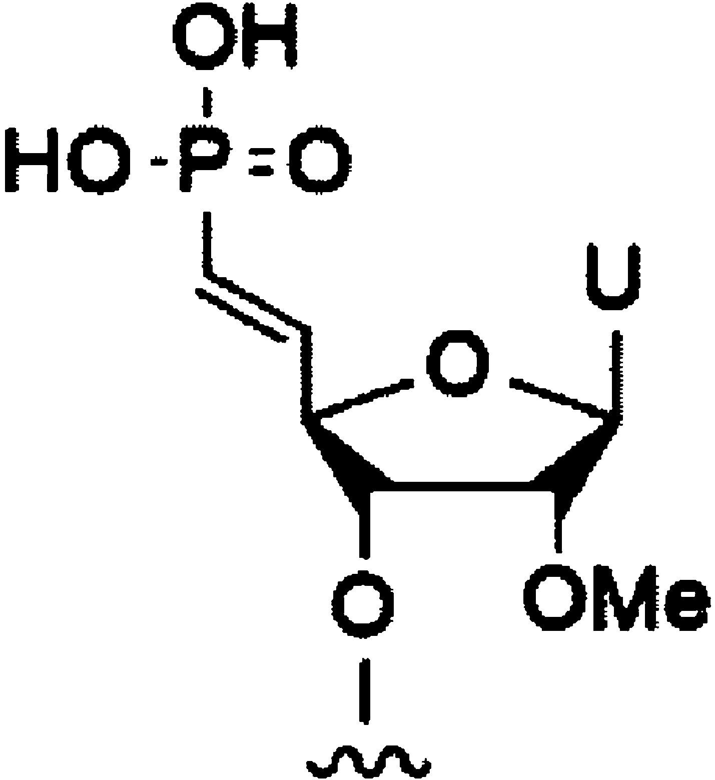 VP- at the 5' end of AS-42 and Um at the end of the oligonucleotide constitute the groups illustrated below, in which the formula: =CH-P(=O)(OH)₂ corresponds to the 5'-end modification (VP-).

17. Use of a pharmaceutical for treatment of a hematologic malignancy, the pharmaceutical including the nucleic acid delivery construct according to claim 1.

18. A method for treating a hematologic malignancy, the method comprising administering the nucleic acid delivery construct according to claim 1 to a patient.

19. A method for inhibiting proliferation of cancerous cells in a hematologic malignancy, the method comprising administering, to the cancerous cells, a nucleic acid delivery construct having an assembly structure in which a nucleic acid analog represented by the following formula (1) and a nucleic acid to be delivered containing a microRNA that regulates a network of the oncogene KRAS are assembled with each other through electrostatic interaction:
[Chem. 11] **L-(S2)s-H-(S1**)t-N **(1**)
wherein N represents a cationic artificial nucleic acid, H represents a hydrophilic polymer, S1 represents a spacer 1, S2 represents a spacer 2, L represents a ligand, s represents 0 or 1, and t represents 0 or 1;
N has structural units in which a base is bonded to a hexose selected from ribose and deoxyribose, linking structures linking two of the structural units, and a backbone structure having cationic groups; and
the cationic artificial nucleic acid is capable of assembling through electrostatic interaction between a phosphate group of the nucleic acid to be delivered and the cationic group.

20. A nucleic acid delivery construct used for treatment of a hematologic malignancy, the nucleic acid delivery construct having an assembly structure in which a nucleic acid analog represented by the following formula (1) and a nucleic acid to be delivered containing a microRNA that regulates a network of the oncogene KRAS are assembled with each other through electrostatic interaction:
[Chem. 12] **L-(S2)s-H-(S1)t-N (1)**
wherein N represents a cationic artificial nucleic acid, H represents a hydrophilic polymer, S1 represents a spacer 1, S2 represents a spacer 2, L represents a ligand, s represents 0 or 1, and t represents 0 or 1;
N has structural units in which a base is bonded to a hexose selected from ribose and deoxyribose, linking structures linking two of the structural units, and a backbone structure having cationic groups; and
the cationic artificial nucleic acid is capable of assembling through electrostatic interaction between a phosphate group of the nucleic acid to be delivered and the cationic group.
